(19) Europäisches Patentamt
European Patent Office
Office européen des brevets

(11) EP 2 821 507 A1

(12) **EUROPEAN PATENT APPLICATION**

(43) Date of publication:
**07.01.2015 Bulletin 2015/02**

(51) Int Cl.:
***C12Q 1/70*** *(2006.01)*

(21) Application number: **13305937.8**

(22) Date of filing: **01.07.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Centre National de la Recherche Scientifique (CNRS)**
  **75016 Paris (FR)**
- **Université de Perpignan Via Domitia**
  **66860 Perpignan Cedex (FR)**

(72) Inventors:
- **Faliex, Elisabeth**
  **66330 Cabestany (FR)**
- **Meistertzheim, Anne-Leïla**
  **66180 Villeneuve-de-la-Raho (FR)**

(74) Representative: **Blot, Philippe Robert Emile**
**Cabinet Lavoix**
**2, place d'Estienne d'Orves**
**75441 Paris Cedex 09 (FR)**

# (54) A method for detecting, identifying and/or quantifying a pathogen in an individual

(57) The present invention concerns methods and kits for detecting, identifying and/or quantifying a pathogen in a tissue sample of an individual, and/or for diagnosing an infection by a pathogen in a tissue sample of an individual, comprising the analysis of high resolution melt curves obtained from cDNA produced from RNA extracted from said tissue sample.

EP 2 821 507 A1

## Description

The present invention concerns methods and kits for detecting, identifying and/or quantifying a pathogen in a tissue sample of an individual, and/or for diagnosing an infection by a pathogen in a tissue sample of an individual, comprising the analysis of high resolution melt curves obtained from cDNA produced from RNA extracted from said tissue sample.

## BACKGROUND OF THE INVENTION

Rhabdoviruses infect a wide range of hosts, including vertebrates, invertebrates and plants. Among vertebrate hosts are a range of fish species from both fresh and marine waters. Historically, fish rhabdoviruses were first assigned to a genus, based on their morphology, protein profiles after gel electrophoresis, and serological cross-reactivity. Classification of several viruses has further evolved with viral genome sequencing, until the creation by the International Committee on Taxonomy of Viruses (ICTV) of the present six genera of the family. Until now all reported rhabdoviruses known to infect fishes belong exclusively to either the *Norvirhabdovirus* or *Vesiculovirus* genus. Unlike the *Norvirhabdovirus* genus, the *Vesiculovirus* genus comprises virus species infecting invertebrates, lower and higher vertebrates.

In 1974, a rhabdovirus was isolated from young American eel *(Anguilla rostrata*) imported from Cuba to Japan and tentatively designated eel virus American (EVA). In 1976 another rhabdovirus was isolated in a shipment of European elvers *(Anguilla anguilla*) from France to Tokyo, which was named eel virus European X (EVEX) because of its European origin. EVA and EVEX are morphologically, serologically, and physicochemically highly similar and regarded as two strains of a single virus species. The classification of EVEX in the *Vesiculovirus* genus was based on electronic microscopy, PAGE protein profile and more recently on complete genome sequence (Galinier et al., 2012). The existence of two lineages with 91.5% sequence identity was confirmed in partial sequences from the RNA polymerase or L gene from EVEX and EVA reference isolates (van Beurden et al., 2011). However, the complete genome of EVA still remains unknown.

Since its first isolation, EVEX has been detected in wild and farmed European eels originating from various geographic regions of its repartition area, such as Italy and France, United Kingdom, the Netherlands, Germany, Denmark and Sweden. EVA has never been reported in European eels; in addition, the susceptibility of European eels for this virus has not been determined experimentally (van Beurden et al., 2011).

EVEX has been identified as a potential agent in the reported decline of *Anguilla anguilla* observed since 1980, as it may become virulent under stressful conditions during aquaculture or during the migration of eels to their spawning area in the Sargasso sea.

Management measures have been established in Europe for the preservation and recovery of the European eel population (Article 7- (CE) 1100/2007)). In this context, France has among others a plan for eel restocking, starting from the early stages of eels (glass eels). These eels are collected, put into breeding and released only if a pool of 10 individuals is virus-free.

A limited number of immunological assays have been described to identify EVEX, such as the enzyme linked immuno-sorbent assay by Dixon and Hill (1984). Using reverse transcriptase and real-time PCR (qPCR), the detection of EVEX was developed in RNA extractions from suspensions of pooled eel organs or EVEX-infected cell cultures showing a cytopathic effect (cpe) during the virological assay (van Beurden et al., 2011). This molecular method detected Dutch EVEX strains and also EVA strain without exact discrimination between the strains except minor differences in amplification efficiency. This molecular tool is still inadequate for precise quantification of virus and discrimination in host tissues. Thus, it is crucial to obtain quantitative and specific tools to determine the prevalence of the virus in wild and farmed eels at individual and tissue level, and also to assess the susceptibility of other eel species to EVEX.

The inventors have created a test, named "EVeel-test", which is the first molecular tool developed for RNA virus quantification in general and for fish rhabdovirus in particular. This test allows the detection, identification and quantification of eel rhabdovirus such as EVA and EVEX strains in a small sample of tissues from a single individual in a single experiment. The main advantages of this novel tool are efficiency, timeliness (1 h30 per assay, compared to over 3 days for the virological test) and low cost (1.40 euros per assay, compared to 60 euros for the virological test). These advantages are major assets for the detection of diseased eels particularly in the context of management measures implemented for the European eel. This tool could for instance be transferred to veterinary organizations already involved in the detection of this virus, or to private laboratories involved in genetic assays, and thus be useful to farmers and European eel responsible farms.

## DESCRIPTION OF THE INVENTION

The inventors have created a new test for specific detection, quantification and identification of pathogens in tissues at individual level in a single, cheap, fast and efficient experiment. This test combines quantitative real time PCR use and High Resolution Melting (HRM) analysis. The assay is composed of several substrates mixed in a sequential manner, starting from RNA extracts from host tissues, reverse-transcribed to produce cDNA, according to a specific procedure. During a DNA amplification step using real-time PCR with HRM analysis, the test directly allows the detection, the identification and the determination of the percentage of pathogen RNA in host RNA from tissues of a single individual.

This tool has been validated individually on separate tissues of different adult eels sampled in different natural populations, including virus-positive populations, as well as glass eel pools. Furthermore, this tool has been validated on samples previously determined as positive by the virological test. The test allowed detection, quantification and discrimination between two closely-related rhabdoviruses, EVEX and EVA.

In one aspect, the invention relates to a method for detecting, identifying and/or quantifying a pathogen in a tissue sample of an individual, and/or for diagnosing an infection by a pathogen in a tissue sample of an individual, said method comprising the steps of:

(a) extracting the RNA from cells of said tissue sample;
(b) producing complementary DNA (cDNA) from the RNA extracted at step (a) by reverse transcription;
(c) amplifying the cDNA produced at step (b) by quantitative real time polymerase chain reaction (qRT-PCR);
(d) performing a High Resolution Melt (HRM) analysis of the qRT-PCR products obtained at step (c);
(e) obtaining a Cycle threshold (Ct) or a Crossing point (Cp) from the qRT-PCR reaction of step (c);
(f) obtaining a melting curve from the HRM analysis results obtained at step (d);
(g) comparing the melting curve obtained at step (f) to a pathogen reference melting curve and/or comparing the Ct or Cp obtained at step (d) to a pathogen or host reference standard curve;
(h) deducing the presence, the identity and/or the quantity of said pathogen in said tissue sample of the individual, and/or deducing that the individual is infected by said pathogen, if the curve obtained at step (f) is similar to said reference curve, and/or the Ct or Cp obtained at step (d) is similar to said reference standard curve.

The invention also pertains to a method for detecting and/or identifying a pathogen in a tissue sample of an individual, said method comprising the steps of:

(a) extracting the RNA from cells of said tissue sample;
(b) producing complementary DNA (cDNA) from the RNA extracted at step (a) by reverse transcription;
(c) amplifying the cDNA produced at step (b) by quantitative real time polymerase chain reaction (qRT-PCR);
(d) performing a High Resolution Melt (HRM) analysis of the qRT-PCR products obtained at step (c);
(e) obtaining a melting curve from the HRM analysis results obtained at step (d);
(f) comparing the melting curve obtained at step (e) to a pathogen reference melting curve;
(g) deducing the presence and/or the identity of said pathogen in said tissue sample of the individual, and/or deducing that the individual is infected by said pathogen, if the curve obtained at step (e) is similar to said reference curve.

The invention further relates to a method for quantifying a pathogen in a tissue sample of an individual, said method comprising the steps of:

(a) extracting the RNA from cells of said tissue sample;
(b) producing complementary DNA (cDNA) from the RNA extracted at step (a) by reverse transcription;
(c) amplifying the cDNA produced at step (b) by quantitative real time polymerase chain reaction (qRT-PCR);
(d) optionally, performing a High Resolution Melt (HRM) analysis of the qRT-PCR products obtained at step (c);
(e) obtaining a Cycle threshold (Ct) or a Crossing point (Cp) from the qRT-PCR reaction of step (c);
(f) optionally, obtaining a melting curve from the HRM analysis results obtained at step (d);
(g) comparing the Ct or Cp obtained at step (e) to a pathogen or host reference standard curve;
(h) deducing the quantity of said pathogen in said tissue sample of the individual based on the comparison at step (g).

Another aspect of the invention concerns a method for diagnosing an infection by a pathogen in a tissue sample of an individual, said method comprising the steps of:

(a) extracting the RNA from the cells of said tissue sample;
(b) producing complementary DNA (cDNA) from the RNA extracted at step (a) by reverse transcription;
(c) amplifying the cDNA produced at step (b) by quantitative real time polymerase chain reaction (qRT-PCR) using primers;
(d) performing a High Resolution Melt (HRM) analysis of the qRT-PCR products obtained at step (c);
(e) obtaining a melting curve from the HRM analysis results obtained at step (d);
(f) comparing the melting curve obtained at step (e) to a reference melting curve of said pathogen;

(g) deducing that the individual is infected by said pathogen if the curve obtained at step (e) is similar to the reference curve.

Preferably, the methods of the invention are in vitro or ex vivo methods.
The pathogen may be, for example, a virus, a bacterium, a fungus, a prion, a protist or a helminth producing RNA. The pathogen may in particular be a rhabdovirus, such as the eel rhabdovirus EVEX or EVA.
The individual according to the invention is preferably an animal, and may in particular be an eel or an elver.
The quantitative real time polymerase chain reaction (qRT-PCR) may be performed using at least one primer having a sequence at least 80, 81, 82, 83, 84, 85, 86, 87, 88, 89, 90, 91, 92, 93, 94, 95, 96, 97, 98, or 99% identical to a sequence selected from the group consisting of SEQ ID NOs: 1 to 18. The qRT-PCR may also be performed using at least one primer capable of hybridizing to a sequence selected from the group consisting of SEQ ID NO: 25 to 36, or sequences complementary thereto, under moderate or high stringency conditions. Alternatively, the qRT-PCR may be performed using at least one primer capable of amplifying a sequence at least 80, 81, 82, 83, 84, 85, 86, 87, 88, 89, 90, 91, 92, 93, 94, 95, 96, 97, 98, or 99% identical to a sequence selected from the group consisting of SEQ ID NO: 19-24. Said primer preferably has features which are optimal for qPCR as described below.
The high resolution melt (HRM) analysis may be performed using at least one dye selected from the group consisting of ResoLight, LC Green, LC Green Plus, Chromofy and SYTO 9. In particular, when absolute quantification is aimed for, the quantification step of a pathogen in a host sample may be performed using at least one dye selected from the group consisting of Resolight and Chromofy.
The reference melting curve or the pathogen or host reference standard curve may be obtained from the EVA "Hill" J6BF4 (GenBank accession number: KC608038), the French EVEX C30 (accession number: JN639009), GG129 (accession number: JN639010) or GG184 (accession number: KC608033), the Dutch EVEX CVI153311 (accession number: FN557213), the German EVEX DF25/04 (accession number: KC608037) or the Danish EVEX DK3545, DK3631 or DK5743 (accession numbers: KC608034, KC608035 and KC608036 respectively).
The present invention further relates to a kit comprising:

- at least one primer comprising or consisting of a nucleic acid sequence at least 80, 81, 82, 83, 84, 85, 86, 87, 88, 89, 90, 91, 92, 93, 94, 95, 96, 97, 98, or 99% identical to a sequence selected from the group consisting of SEQ ID NO: 1 to 18; and/or
- optionally, at least one reference melting curve obtained from the EVA "Hill" J6BF4 (GenBank accession number: KC608038), the French EVEX C30 (accession number: JN639009), GG129 (accession number: JN639010) or GG184 (accession number: KC608033), the Dutch EVEX CVI153311 (accession number: FN557213), the German EVEX DF25/04 (accession number: KC608037) or the Danish EVEX DK3545, DK3631 or DK5743 (accession numbers: KC608034, KC608035 and KC608036 respectively);
- optionally, at least one control sample of known concentration of nucleic acid from EVA "Hill" J6BF4 (GenBank accession number: KC608038), French EVEX C30 (accession number: JN639009), GG129 (accession number: JN639010) or GG184 (accession number: KC608033), Dutch EVEX CVI153311 (accession number: FN557213), German EVEX DF25/04 (accession number: KC608037), or the Danish EVEX DK3545, DK3631 or DK5743 (accession numbers: KC608034, KC608035 and KC608036 respectively); and
- optionally, at least one control sample of known concentration of nucleic acid of reference genes from a host
- optionally, instructions for use.

<u>Definitions</u>

As used herein, the step of <u>extracting the RNA</u> from cells refers to any molecular biological method permitting isolation of RNA from samples.
The step of <u>producing complementary DNA</u> (cDNA) from the extracted RNA refers to synthesis of cDNA from the extracted RNA used as a template. Said synthesis may for instance be performed by using a reverse transcriptase enzyme, in the presence of random primers and of deoxynucleotide triphosphates (A, T, G, C), for instance hexamer primers. This enzyme synthesizes one complementary strand of DNA hybridized to the initial RNA strand..
The step of <u>amplifying the</u> cDNA may be performed by quantitative real time polymerase chain reaction (qRT-PCR). As used herein, the terms <u>quantitative real time polymerase chain reaction</u> (qRT-PCR), <u>real-time polymerase chain reaction (RT-PCR), quantitative real-time polymerase chain reaction (qPCR)</u> or <u>kinetic polymerase chain reaction</u> are equivalent, and refer to a molecular biology technique based on PCR, which allows amplifying and simultaneously quantifying one or more specific sequences in a DNA sample.
Specific sequences present in the cDNA sample may be selectively amplified using specific primers, *i.e.* primers that specifically hybridize to particular sequences present in the cDNA sample. For instance, primers that specifically hybridize to sequences selected from the group consisting of SEQ ID NO: 25 to 36, or sequences complementary thereto, under moderate or high stringency conditions may be used. SEQ ID NO: 1 to 18 are examples of such primers.

Primers for DNA amplification preferably have features which are optimal for qPCR. Such features include an optimal primer length of 20 bases, hybridization temperature between 58°C and 60°C with optimal at 59°C, length of amplicon product from 80 to 200 bases, percentage of GC in primers between 30 to 80%, with the last five nucleotides at the 3' end containing no more than two G + C residues. Other desirable features include avoiding repeating oligonucleotides of more than four consecutive G residues, avoiding secondary structures (hairpin loops, self-dimerization and cross-dimerization and non-specific hybridization, including in host, bacterium or virus genome). Primers should be localized in conserved areas of all the virus strains and flank the region of variability of all the virus strains.

<u>High stringency hybridization conditions</u> may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/5 OmM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42 °C in 0.2 x SSC (sodium chloride/sodium citrate) and 50% formamide at 55°C, followed by a high-stringency wash consisting of 0.1 x SSC containing EDTA at 55°C. Moderately stringent conditions may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C.

Alternatively, primers may also be chosen to specifically amplify regions corresponding to specific loci in the pathogen RNA. For instance, primers capable of amplifying a sequence at least 80, 81, 82, 83, 84, 85, 86, 87, 88, 89, 90, 91, 92, 93, 94, 95, 96, 97, 98, or 99% identical to a sequence selected from the group consisting of SEQ ID NO: 19-24 may be used.

<u>Nucleic acid sequence identity</u> is defined as the percentage of nucleic acids in the variant sequence that are identical with the nucleic acids in the reference sequence, after aligning the sequences and introducing gaps, if necessary, to achieve the maximum percent sequence identity, and not considering any conservative substitutions as part of the sequence identity. Sequence identity may be determined over the full length of the variant sequence, the full length of the reference sequence, or both.

Methods for sequence alignment and determination of sequence identity are well known in the art, for example using publicly available computer software such as BioPerl, BLAST, BLAST-2, CS-BLAST, FASTA, ALIGN, ALIGN-2, LALIGN, Jaligner, matcher or Megalign (DNASTAR) software and alignment algorithms such as the Needleman-Wunsch and Smith-Waterman algorithms.

For example, the percentage identity may be calculated by performing a pairwise global alignment based on the Needleman-Wunsch alignment algorithm to find the optimum alignment (including gaps) of two sequences along their entire length, for instance using Needle, and using the BLOSUM62 matrix with a gap opening penalty of 10 and a gap extension penalty of 0.5.

When studying nine EVEX and EVA strains, the inventors have shown that for each strain a specific melting curve was observed at least for one locus, except for the EVEX C30 strain (see Table 3). For instance, primers specifically amplifying the N, P, G, La or Lb loci may be used to identify the EVEX CVI153311 strain (accession number: FN557213). Primers specifically amplifying the P, G, or Lb loci may be used to identify the EVEX GG129 strain (accession number: JN639010). Primers specifically amplifying the G locus may be used to identify the EVEX GG184 strain (accession number: KC608033). Primers specifically amplifying the P locus may be used to identify the EVEX DF25/04 strain (accession number: KC608037). Primers specifically amplifying the La locus may be used to identify the EVEX DK3545 strain (accession number: KC608034). Primers specifically amplifying the P, La, or Lb locus may be used to identify the EVEX DK3631 strain (accession number: KC608035). Primers specifically amplifying the Lb locus may be used to identify the EVEX DK5743 strain (accession number: KC608036). Primers specifically amplifying the N, M, G, La or Lb locus may be used to identify the EVA "Hill" J6BF4 strain (GenBank accession number: KC608038). The EVEX C30 strain (accession number: JN639009) may be identified by subtracting the group of strains identified by amplifying the N locus (EVEX C30, EVEX DK3545 and EVEX 5743 strains) with the groups of strains identified by amplifying the La and Lb loci (group of EVEX DK5743 and C30, and group of EVEXDK3545 and C30 strains, respectively).

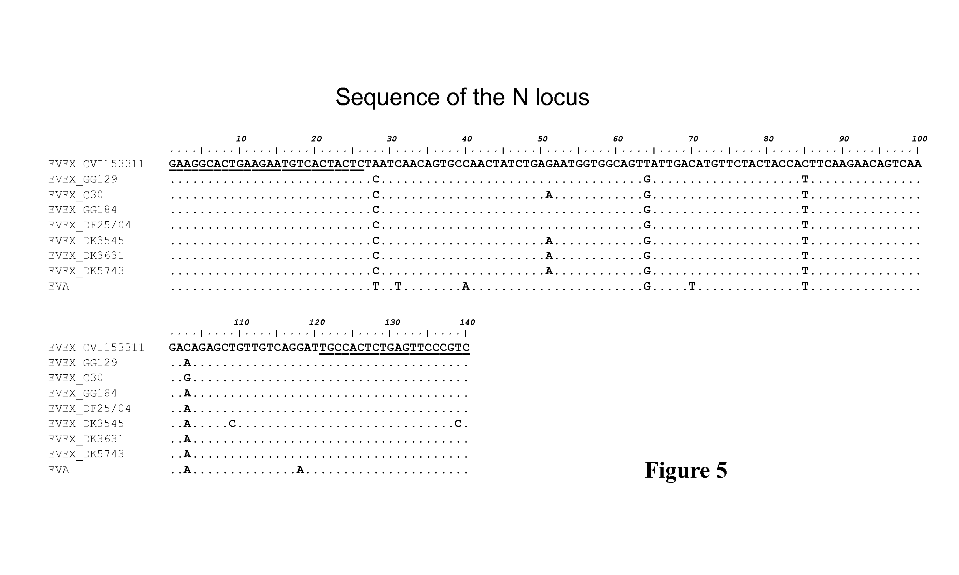

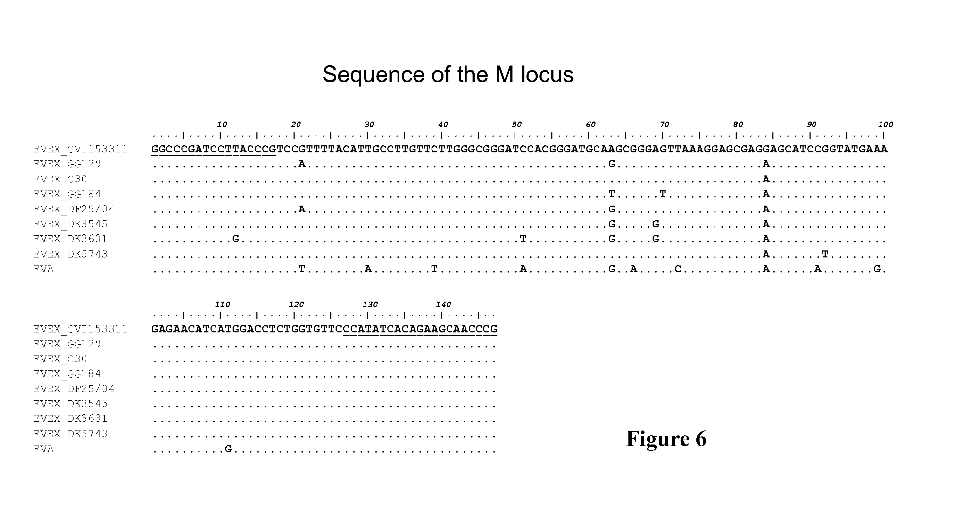

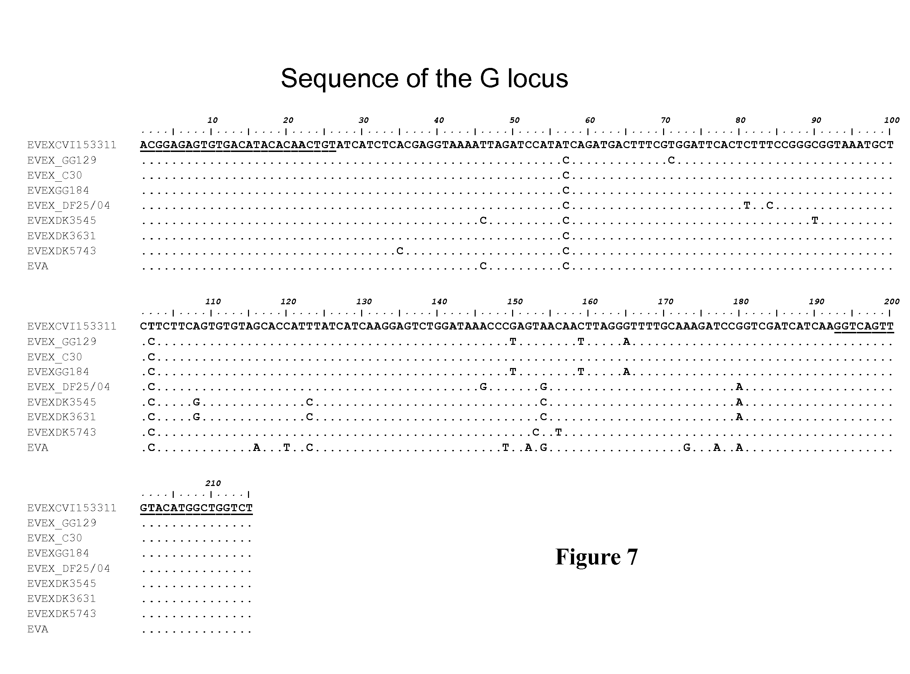

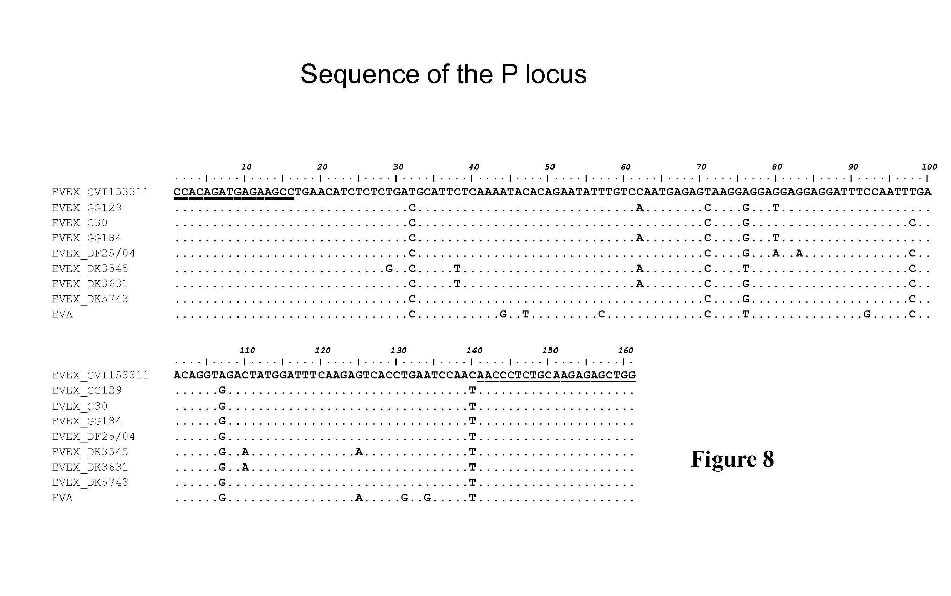

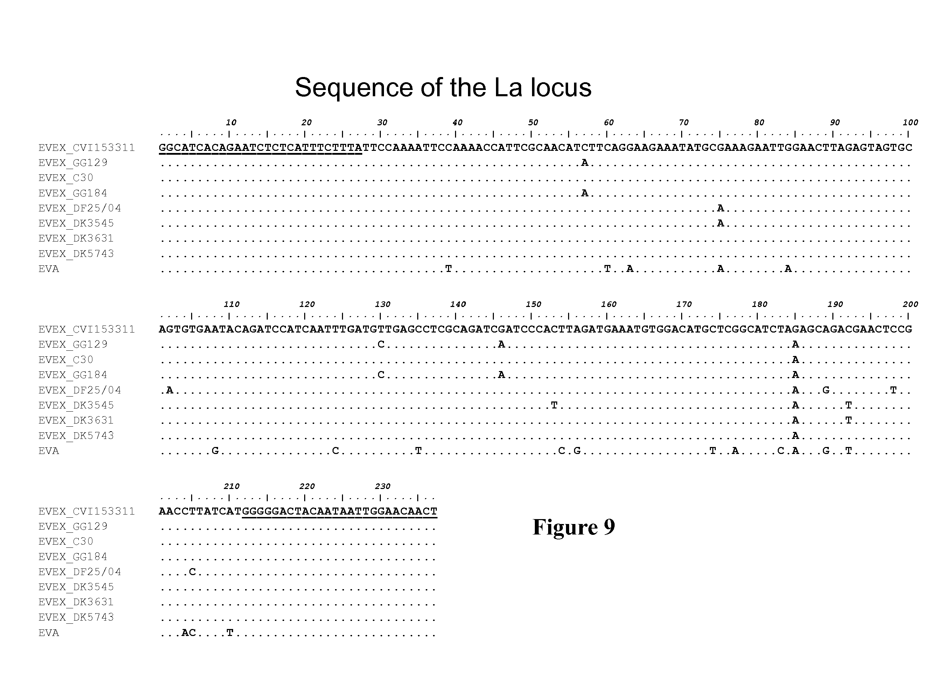

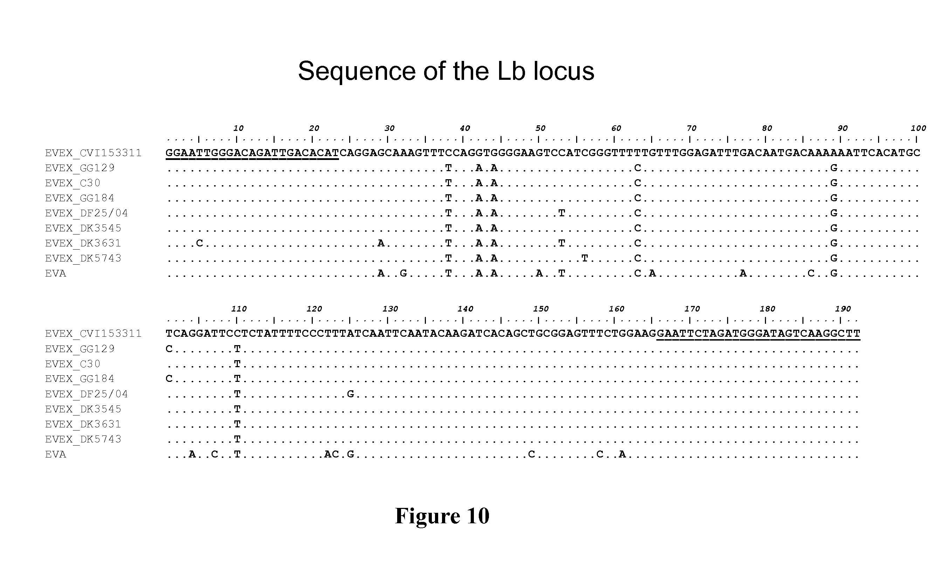

The inventors have designed specific primers for the amplification of the N, P, M, G, La or Lb locus of the rhabdovirus EVEX and EVA strains (see sequences SEQ ID NO: 1 to 18), as well as for the amplification of three housekeeping eel genes. Thus, in some embodiments, the qRT-PCR reaction may also be performed using at least one primer having a sequence at least 80, 81, 82, 83, 84, 85, 86, 87, 88, 89, 90, 91, 92, 93, 94, 95, 96, 97, 98, or 99% identical to a sequence selected from the group consisting of SEQ ID NO: 1 to 18. In some embodiments, the pRT-PCR reaction may be performed using at least one primer having a sequence complementary to a portion of SEQ ID NO: 19 to 24 or 37 to 84. Preferably, said portion of SEQ ID NO: 19 to 24 or 37 to 84 lies within a region which is common to all or most virus strains, as shown in Figures 5 to 10. Preferably, said portion of SEQ ID NO: 19 to 24 or 37 to 84 flanks a region which variable between all or most virus strains, as shown in Figures 5 to 10. For example, said portion may lie within the region corresponding to amino acids 1 to 27 or 119 to 140 of SEQ ID NO: 19; the region corresponding to amino acids 1 to 20 or 112 to 147 of SEQ ID NO: 20; the region corresponding to amino acids 1 to 34 or 181 to 205 of SEQ ID NO: 21; the region corresponding to amino acids 1 to 28 or 141 to 161 of SEQ ID NO: 22; the region corresponding to amino acids 1 to 37 or 211 to 237 of SEQ ID NO: 23; or the region corresponding to amino acids 1 to 28 or 162 to 192 of SEQ ID NO: 24.

Said primer may be at least 5, 6, 7, 8, 9, 10, 11, 12, 13, 14, 15, 16, 17, 18, 19, 20, 21, 22, 23, 24, 25, 26, 27, 28, 29 or 30 nucleotides in length, and/or not more than 10, 11, 12, 13, 14, 15, 16, 17, 18, 19, 20, 21, 22, 23, 24, 25, 26, 27, 28, 29, 30, 31, 32, 33, 34 or 35 nucleotides in length.

Another aspect of the invention concerns a kit comprising at least one primer as described herein.

The step of detecting and/or analyzing the amplified cDNA may be performed as the PCR reaction progresses in real time. In the context of the invention, the step of analyzing the amplified cDNA is performed by high resolution melt analysis.

As used herein, the terms High Resolution Melt, Hi-Res Melting, or HRM are equivalent. HRM analysis may be performed using non-specific fluorescent dyes that specifically bind to double-stranded (ds) DNA, causing fluorescence of the dye. In some embodiments, at least one dye selected from the group consisting of ResoLight, LC Green, LC Green Plus, Chromofy and SYTO 9 is used in the method of the invention. When absolute quantification is aimed for, in particular in the method for quantifying a pathogen according to the invention, the quantification step of a pathogen in a host sample may be performed using at least one dye selected from the group consisting of Resolight and Chromofy.

At the beginning of the HRM analysis there is a high level of fluorescence in the sample because of the presence of the newly amplified double-stranded cDNA. During HRM analysis, the sample is heated up, the qRT-PCT products are progressively denaturized, the two strands of the DNA melt apart, presence of double stranded DNA decreases and thus fluorescent signal decreases. Fluorescence intensity may thus be measured at each cycle, allowing DNA concentrations to be quantified. The quantity can be either an absolute number of copies or a relative amount when normalized to DNA input or additional normalizing molecules. Thus, in a specific embodiment, the method of the invention is a method for quantifying a pathogen in a tissue sample of an individual.

A real-time PCR machine, coupled with HRM analysis may be used, such as e.g. Roche's LightCycler 480, Applied Biosystems 7500 Fast System and 7900HT Fast Real-Time PCR System, Stratagene Mx3005, or Qiagen's Rotor-Gene instruments. Such a machine has a camera that watches the HRM process by measuring the fluorescence level with a detector. The machine may also plot the fluorescence intensity data as a graph known as a "melt curve" and calculate the Cycle threshold (Ct) or Crossing point (Cp) using absolute quantification.

In the context of the invention, the terms "Cycle threshold" (Ct) and "Crossing point" (Cp) are equivalent and reflect the cycle number at which the fluorescence generated within a reaction crosses the threshold. The Ct value is inversely correlated to the logarithm of the initial copy number.

As used herein, the terms melt curve and melting curve are equivalent and refer to a curve showing the level of fluorescence as a function of the temperature. The melting temperature (Tm) corresponds to the temperature at which half of the double strand DNA is denatured. In the context of the invention, the term melting curve may also refer to the graph of the negative first derivative of fluorescence as a function of the temperature (-dF/dT) which may render easier determining the melting temperature (Tm) of each PCR product, by virtue of the peak thus formed.

Each PCR product may be identified by its melting temperature (Tm). HRM analysis may detect single nucleotide polymorphisms (SNPs), small insertions or deletions, in a fragment of amplified DNA by comparing the obtained melting curve to a reference melting curve.

As used herein, a reference melting curve may correspond to a single value, such as e.g. the Tm, or a range of values which is determined based on the fluorescence level measured following amplification of nucleic acids of one or more given pathogen(s), optionally at different concentrations and/or mixed with host nucleic acids, and HRM analysis thereof. The reference melting curve may be pre-determined prior to implementation of the method of the invention. Alternatively, the reference melting curve may be generated simultaneously to implementation of the method of the invention by adding RNA from one or more pathogen(s) at the step of producing cDNA from RNA by reverse transcription or by adding cDNA from one or more pathogen(s) at the step of amplifying the cDNA by qRT-PCR, which shall be used as a positive control of the presence of said pathogen(s) in the sample.

The invention further pertains to a method for quantifying a pathogen which may comprise quantification of RNA from the pathogen and host in a sample of RNA, using host and pathogen reference standard curves. As used herein, a pathogen, respectively host, reference standard curve may correspond to a single value, or a range of values which is determined based on the fluorescence level measured following amplification of nucleic acids of one or more given pathogen(s), respectively host(s), optionally at different concentrations.

When absolute quantification is aimed for, in particular in the method for quantifying a pathogen according to the invention, the pathogen reference standard curve may be generated simultaneously to implementation of the method of the invention by adding RNA from one or more pathogen(s) at a known concentration at the step of producing cDNA from RNA by reverse transcription, or by adding cDNA from one or more pathogen(s) at a known concentration at the step of amplifying the cDNA by qRT-PCR, which shall be used as a positive control of the presence of said pathogen(s) in the sample, as well as a reference standard curve for pathogen quantification after serial dilution.

Quantification of host RNA may also be performed during implementation of the method of the invention, by adding RNA from the host at a known concentration at the step of producing cDNA from RNA by reverse transcription or cDNA from the host at a known concentration at the step of amplifying the cDNA by qRT-PCR, which may then be used to generate a host reference standard curve for RNA quantification.

The reference standard curves may be obtained from loci synthesis, viral cultures or uninfected host samples.

According to some embodiments, the reference melting curve or the pathogen reference standard curve is obtained from one or more of the following pathogen: the EVA "Hill" J6BF4 (GenBank accession number: KC608038), the French EVEX C30 (accession number: JN639009), GG129 (accession number: JN639010) or GG184 (accession number: KC608033), the Dutch EVEX CVI153311 (accession number: FN557213), the German EVEX DF25/04 (accession number: KC608037) or the Danish EVEX DK3545, DK3631 or DK5743 (accession numbers: KC608034, KC608035 and KC608036 respectively). Preferably, the reference melting curve or the pathogen reference standard curve is obtained by amplifying one or more of the N, P, G, La or Lb loci of an EVEX or an EVA strain. More preferably, the reference melting curve or the pathogen reference standard curve is obtained by amplifying one or more sequence(s) at least 80, 81, 82, 83, 84, 85, 86, 87, 88, 89, 90, 91, 92, 93, 94, 95, 96, 97, 98, or 99% identical to a sequence selected from the group consisting of SEQ ID NO: 19-24.

An aspect of the invention concerns a kit comprising at least one reference melting curve obtained from the EVA "Hill" J6BF4 (GenBank accession number: KC608038), the French EVEX C30 (accession number: JN639009), GG129 (accession number: JN639010) or GG184 (accession number: KC608033), the Dutch EVEX CVI153311 (accession number: FN557213), the German EVEX DF25/04 (accession number: KC608037) or the Danish EVEX DK3545, DK3631 or DK5743 (accession numbers: KC608034, KC608035 and KC608036 respectively).

The comparison of the melting curve obtained with the sample and the reference melting curve obtained from a known pathogen allows the presence of said known pathogen in the sample to be determined: if the melting curve obtained with the sample and the reference melting curve obtained from a known pathogen are similar, then it may be deduced that said known pathogen is present in the sample. Thus, in a specific embodiment, the method of the invention is a method for detecting a pathogen in a tissue sample of an individual.

Curves may be considered as <u>similar</u> if they are substantially superimposable or if their values are very close. Values may be considered as similar if they differ by less than 0.2, 0.1, or preferably 0.01 units. For instance, Tm values may be considered as similar if they differ by less than 0.2, 0.1, or preferably 0.01 °C.

The reference melting curve may also be obtained from a pool of known pathogens or strains of pathogens, optionally at different concentrations and/or mixed with host nucleic acids. Thus, in a specific embodiment, the method of the invention is a method for identifying a pathogen in a tissue sample of an individual, by comparing the melting curve obtained from the sample to the reference melting curve obtained from a pool of known pathogens or stains of pathogens. The invention also relates to a method for diagnosing an infection by a pathogen in a tissue sample of an individual.

The methods according to the invention allow the detection, identification and/or quantification of a pathogen in a tissue sample of an individual.

As used herein, the term <u>pathogen</u> refers to any kind of pathogen. For instance, the pathogen may be a RNA virus, or a pathogen producing RNA, such as for instance aDNA virus, a bacterium, a fungus, a prion, a protist, a helminth etc. Preferably, the pathogen is a virus. More preferably, the pathogen is a rhabdovirus. Even more preferably, the pathogen is a rhabdovirus of eel, such as for instance an EVEX or an EVA strain.

As used herein, the term <u>EVA</u> refers to "eel virus American", a rhabdovirus originally isolated from young American eel *(Anguilla rostrata*) imported from Cuba to Japan. The term may for instance refer to the EVA "Hill" J6BF4 strain (GenBank accession number: KC608038).

As used herein, the term <u>EVEX</u> refers to "eel virus European X", a rhabdovirus originally isolated in a shipment of European elvers *(Anguilla anguilla*) from France to Tokyo. Several strains of EVEX are known among which the French EVEX C30 (accession number: JN639009), GG129 (accession number: JN639010) or GG184 (accession number: KC608033), the Dutch EVEX CVI153311 (accession number: FN557213), the German EVEX DF25/04 (accession number: KC608037) or the Danish EVEX DK3545, DK3631 or DK5743 (accession numbers: KC608034, KC608035 and KC608036 respectively).

In the context of the invention, the <u>individual</u> may be any kind of cellular organism, such as a plant or an animal. In a specific embodiment, the individual is an animal. Preferably, the individual is an eel.

As used herein, the term <u>host</u> refers to a group of organisms which is potentially infected by a pathogen. In the context of the method for detecting, identifying and/or quantifying a pathogen in a tissue sample of an individual, and/or for diagnosing an infection by a pathogen in a tissue sample of an individual, the term host designates the group of organisms to which the individual belongs. The host may be any kind of cellular organism, such as a plant or an animal. In a specific embodiment, the host is an animal. Preferably, the host is an eel.

The tissue sample of the individual may be a sample from any kind of tissue. When the individual is an animal, the tissue sample may for instance come from kidney, spleen, heart or brain, or be a mixture of tissues from various organs. When the individual is a small animal, for instance an elver, the tissue sample to be used in the method may comprise samples from tissues of the whole body of the animal.

The present invention further relates to a kit comprising at least one primer comprising or consisting of a nucleic acid sequence at least 80, 81, 82, 83, 84, 85, 86, 87, 88, 89, 90, 91, 92, 93, 94, 95, 96, 97, 98, or 99% identical to a sequence selected from the group consisting of SEQ ID NO: 1 to 18.

The kit according the invention may also comprise one or more reference melting curve(s) and/or control sample(s) of known concentration. For instance, the kit may comprise a control sample of known concentration of nucleic acids of reference genes from a host. In a specific embodiment, the host is an eel, in particular *A. Anguilla,* and reference genes are the 60S acidic ribosomal protein p0 (eel_rep_c57236, EeelBase), the ribosomal protein L22 (eeel2_c1866, EeelBase) and the ribosomal protein s18 (eeel2_s7245, EeelBase).

## BRIEF DESCRIPTION OF THE SEQUENCES

SEQ ID NO: 1-18 show the sequences of primers (see Table 1 for details).
SEQ ID NO: 19-24 show the reference sequences of the N, M, G, P, La and Lb loci of the EVEX CVI153311 respectively.
SEQ ID NO: 25-36 show the sequences of the N, M, G, P, La and Lb loci of the EVEX and EVA strains to which the primers according to the invention may hybridize.
SEQ ID NO: 37-42 show the reference sequences of the N, M, G, P, La and Lb loci of the EVEX GG129 respectively.
SEQ ID NO: 43-48 show the reference sequences of the N, M, G, P, La and Lb loci of the EVEX C30 respectively.
SEQ ID NO: 49-54 show the reference sequences of the N, M, G, P, La and Lb loci of the EVEX GG184 respectively.
SEQ ID NO: 55-60 show the reference sequences of the N, M, G, P, La and Lb loci of the EVEX DF25/04 respectively.
SEQ ID NO: 61-66 show the reference sequences of the N, M, G, P, La and Lb loci of the EVEX DK3545 respectively.
SEQ ID NO: 67-72 show the reference sequences of the N, M, G, P, La and Lb loci of the EVEX DK3631 respectively.
SEQ ID NO: 73-78 show the reference sequences of the N, M, G, P, La and Lb loci of the EVEX DK5743 respectively.
SEQ ID NO: 79-84 show the reference sequences of the N, M, G, P, La and Lb loci of the EVA respectively.

## BRIEF DESCRIPTION OF THE FIGURES

**Figure 1: Melting curves obtained for the 8 EVEX and 1 EVA strains at the loci N and Lb.** HRMA detects mutations in DNA fragments due to temperature shift of the melting curve caused by variation of the amplicon Tms. The amplicon Tms at locus N showed a difference of 2°C between EVA and EVEX strains, and a difference of 0.5°C at locus Lb between EVEX strains.

**Figure 2: Melting curves obtained for the 8 EVEX and 1 EVA strains using EVEX.F04 and EVEX.R04 previously developed by van Beurden *et al*., 2011.** The amplicon Tms showed a difference of 2°C between EVEX DF27/04, CV153311 and the other strains, including EVA strain.

**Figure 3: Normalized melting peaks at locus N of EVA and EVEX standards mixed at different proportions.** EVA and EVEX GG129 melting peaks appeared at 80 and 82°C, respectively, for all mixed virus standards.

**Figure 4: Amplification at locus N.** Standard curves of virus RNA transcript standards were linear for viral RNA values from $5.10^{-3}$ to $5.10^{2}$ ng/reaction corresponding to a range of viral RNA from $10^{-5}$ to 1 ng/TotalRNAng. Normalization of values per three host reference genes (NRQ) was also linear in this range.

**Figures 5 to 10: Alignment of the sequences of the N, M, G, P, La and Lb loci (respectively) for the different EVEX and EVA strains.**

**Table 1. Combinations of forward and reverse primers used in real-time PCR and High Resolution analyses.**

| Locus name | Forward primer (5'→3') | SEQ ID NO: | Reverse primer (5'→3') | SEQ ID NO: | PCR efficiency (E) |
|---|---|---|---|---|---|
| ***Viral genes*** | | | | | |
| N | GAAGGCACTGAAGAATGTCACTACTC | SEQ ID NO: 1 | GACGGGAACTCAGAGTGGCA | SEQ ID NO: 10 | 1.91 |
| M | GGCCCGATCCTTACCCG | SEQ ID NO: 2 | CGGGTTGCTTCTGTGATATGG | SEQ ID NO: 11 | 1.99 |
| G | ACGGAGAGTGTGACATACACAACTGT | SEQ ID NO: 3 | AGACCAGCCATGTACAACTGACC | SEQ ID NO: 12 | 1.97 |
| P | CCACAGATGAGAAGCCTGAACAT | SEQ ID NO: 4 | CCAGCTCTCTTGCAGAGGGTT | SEQ ID NO: 13 | 1.93 |
| La | GGCATCACAGAATCTCTCATTTCTTTA | SEQ ID NO: 5 | CAGTTGTTCCAATTATTGTAGTCCCC | SEQ ID NO: 14 | 1.91 |
| Lb | GGAATTGGGACAGATTGACACAT | SEQ ID NO: 6 | AAGCCTTGACTATCCCATCTAGAATTC | SEQ ID NO: 15 | 1.85 |
| ***Ribosomal proteins*** | | | | | |
| 60S p0 | ATGGAGGCAAGAGTTGGGTATC | SEQ ID NO: 7 | TGGAGGGTGTGAGGAATATCG | SEQ ID NO: 16 | 1.99 |
| L22 | TGGCAGGGCTGGAAACC | SEQ ID NO: 8 | CCAGTCTCTCAGATTGTTCTTTTTCAG | SEQ ID NO: 17 | 1.92 |
| s18 | ATACCGCAGCTAGGAATAATG | SEQ ID NO: 9 | GGCCGTCCCTCTTAATCA | SEQ ID NO: 18 | 1.92 |

**Table 2. Examples of linear equations derived from standard curves obtained for the loci N, Lb and using the primer pair EVEX.F04/EVEX.R04.** Consensus equations were calculated for the 8 EVEX strains (named EVEX Consensus) and for all the 9 strains including EVA (named Total Consensus). Consensus linear equations were used to calculate the quantities of viral RNA in field samples.

| **Locus** | **N** | | | | | | **Lb** | | | | | | **EVEX.F04/EVEX.R04 (van Beurden et al., 2011)** | | | | | |
|---|---|---|---|---|---|---|---|---|---|---|---|---|---|---|---|---|---|---|
| **y** | **NRQ** | | | **Ct** | | | **NRQ** | | | **Ct** | | | **NRQ** | | | **Ct** | | |
| y=a.log($RNA_{ng}$)+ b | a | b | $R^2$ | a | b | $R^2$ | a | b | $R^2$ | a | b | $R^2$ | a | b | $R^2$ | a | b | $R^2$ |
| C30 | 1.47 | -2.93 | 0.99 | -4.57 | 33.52 | 0.98 | 1.56 | -3.12 | 0.95 | -5.13 | 32.98 | 0.98 | 1.43 | -2.85 | 0.99 | -4.51 | 32.93 | 0.99 |
| DF25/04 | 1.50 | -4.22 | 0.90 | -4.74 | 30.97 | 0.91 | 1.52 | -4.15 | 0.90 | -5.04 | 30.50 | 0.91 | -1.58 | 2.76 | 0.49 | -6.23 | 31.62 | 0.78 |
| GG129 | 1.53 | -3.80 | 1.00 | -3.91 | 29.10 | 1.00 | 1.51 | -3.74 | 1.00 | -4.11 | 28.01 | 1.00 | 1.57 | -3.89 | 1.00 | -4.14 | 28.84 | 1.00 |
| *EVEX Consensus* | 1.64 | -3.94 | **0.89** | -4.60 | 31.38 | **0.89** | 1.63 | -3.91 | **0.90** | -5.00 | 30.86 | **0.90** | -1.57 | 2.09 | 0.45 | -5.93 | 32.63 | 0.74 |
| EVA | 1.07 | -2.71 | **1.00** | -3.31 | 30.98 | **0.99** | 1.66 | -3.38 | **0.87** | -5.54 | 33.11 | **0.87** | -2.67 | 5.94 | 0.61 | -3.41 | 42.06 | 0.93 |
| *Total Consensus* | 1.31 | -3.34 | **0.83** | -4.02 | 30.98 | **0.90** | 1.60 | -3.70 | **0.90** | -5.39 | 31.86 | **0.88** | -2.32 | 3.84 | 0.52 | -3.52 | 32.24 | 0.09 |

**Table 3. Differentiation pattern between EVEX and EVA strains obtained using the 6 loci** of **the EVeel-Test.** In each column, a similar symbol indicates the same melting curve pattern (white symbols). For each strain, a specific melting curve was observed at least for one locus, indicated by a specific black symbol.

| | Locus | | | | | |
|---|---|---|---|---|---|---|
| **Souche** | N | P | M | G | La | Lb |
| EVEX CVI153311 | ♦ | ♦ | □ | ♦ | ♦ | ♦ |
| EVEX GG129 | ○ | ▲ | ○ | ▲ | □ | ▲ |
| EVEX GG184 | ○ | □ | ○ | ► | □ | ○ |
| EVEX C30 | □ | ○ | ○ | ○ | ○ | ○ |
| EVEX DF25/04 | ○ | ◄ | ○ | □ | □ | ○ |
| EVEX DK3545 | □ | ○ | ○ | ○ | — | ○ |
| EVEX DK3631 | ○ | ● | □ | □ | ● | ● |
| EVEX DK5743 | □ | ○ | ○ | ○ | ○ | ▼ |
| EVA J6BF4 | ■ | □ | ■ | ■ | ■ | ■ |

**EXAMPLES**

***Materials and Methods***

**Example 1: Field collection and virus cellular detection**

Sixty live yellow and silver eels *(Anguilla anguilla*) (average total length = 35 cm) were collected between September 2009 and 2011 in Salses-Leucate lagoon, on the French north-west Mediterranean coast. Tissues were dissected and immediately frozen in liquid nitrogen. Detection of EVEX virus was preliminarily carried out using the unique cellular test detection, requiring individual pool of organs including kidney, spleen, heart and brain. Detection or EVEX virus was also carried out in 16 pools of 10 glass eels each, collected in Atlantic French estuaries, homogenized in culture medium and frozen. The tissues were homogenized in Buffer and routinely processed for virological examination in epithelioma papulosum cyprini (EPC), rainbow trout gonad (RTG2) and bluegill fry (BF2) cells, as described in Fijan et al. 1983, Wolf et al. 1966, and Wolf and Quimby 1962, at both 14°C and 20°C by the Agence Nationale de Sécurité Sanitaire de l'alimentation, de l'environnement et du travail (ANSES, Plouzané, France).

**Example 2: Virus strains**

EVA (Eel Virus American) was isolated in 1974 from an epizootic of hemorrhagic disease in elvers imported into Japan from Cuba. EVA is an eel rhabdovirus isolated from American eel *(Anguilla rostrata*) using RTG-2 cells as described in Sano, 1976. The virus was obtained by the Cefas Weymouth Laboratory from T. Sano (Tokyo University of Fisheries). The isolate of this strain was previously described in Sano, 1976, and a frozen aliquot of the original EVA "Hill" J6BF4 was used for complete genome sequence determination.

The EVEX C30 strain used by the inventors was originally isolated in 1976 from European elvers originating from the French Atlantic coast. EVEX isolate CVI153311 was isolated from farmed eel in the Netherlands in 1992. The EVEX DF25/04 was isolated in 2004 from glass eels originating from the German Atlantic coast. EVEX DK3545, DK3631 and DK5743 were isolated in 1986 and 1990 from eels originating from Denmark. EVEX GG129 and GG184 were isolated in November 2008 from a pool of organs including kidney, spleen, brain and heart from two European silver eels originating from Salses-Leucate lagoon, on the French north-west Mediterranean coast. The isolation of some strains were previously described (Galinier et al., 2012), and a frozen aliquot of the original C30, GG129, GG184, CVI153311 were used for virus assays. The French EVEX GG184, German EVEX DF25/04 and Danish EVEX DK3545, DK3631 and DK5743 were not previously described and used for genome sequencing.

**Example 3: RNA extraction, RT-PCR and new strains sequencing**

Viral RNA was isolated from frozen supernatants of viral culture medium. Host RNA was extracted from kidney, spleen, heart and brain of yellow eels individually and from pooled glass eels in culture medium. RNA was isolated using TRIzol® Reagent (Invitrogen, Carlsbad, CA) according to the manufacturer's instructions after homogenization at a ratio of 1 mL Trizol to 200 μL of supernatant or 50 mg of tissue, using a tissue tearer, (Ultrathurax) on ice. RNAs were resuspended in RNase-free water and their quantity was assessed by spectrophotometry (Nanodrop ND-1000, Thermo Fisher scientific, USA). Reverse transcription was performed with 500 ng of RNA from each sample using hexamer primers and RevertAid™ kit (Fermentas, International Inc., Burlington, Canada). A set of EVEX primers were designed and used to amplify the entire genome of EVA, German EVEX DF25/04, Danish EVEX DK3545, DK3631 and DK5743 and French EVEX GG184. All the PCRs were performed with the Advantage 2 PCR system according to the manufacturer's instructions (Invitrogen, Carlsbad, CA, USA). Each amplified fragment was sent for sequencing (Genoscreen, Lille, France).

**Example 4: Analysis of the nucleotide and deduced amino acid sequences**

The Seqscape v2.5 program (Applied Biosystems) was used to assemble DNA fragments. Alignments of nucleotide sequences of all the virus isolates were performed using BioEdit freeware version 7.0.9 (Ibis Biosciences, Carlsbad, CA 92008, USA). The complete genome sequence of the German EVEX isolate DF25/04 has been deposited in the EMBL nucleotide sequence database under accession number KC608037, the Danish EVEX DK5743, DK3545, DK3631 isolates have been deposited under the accession numbers KC608036, KC608034 and KC608035, respectively, the French EVEX isolate GG184 has been deposited under the accession number (GenBank: KC608033) and the Japanese EVA isolate has been deposited under the accession number KC608038.

**Example 5: Preparation of standard curves for qPCR quantification**

To quantify the virus concentration (viral RNA and its RNA transcripts), standard curves were created with the nine EVEX and EVA virus strains at different concentrations. Each standard curve was made in duplicate to determine the assay precision. Total RNA of each virus was extracted as described above starting from several frozen aliquots of cell culture supernatants. RNA from three uninfected hosts (negative virological examination, ANSES) was extracted from pooled tissues (brain, kidney, spleen, and heart). Standard curves were designed by adding a range of viral RNA (from $5.10^{-8}$ to $5.10^{2}$ ng) to host RNA to reach a total of 500 ng for reverse transcription (described above).

**Example 6: qPCR and High Resolution Melting Analysis**

A fragment of the five viral genes and three reference sequences encoding ribosomal protein genes from the host were amplified by real time PCR (qPCR) using primers designed by Primer Express software (see Table 1 hereafter). Primers previously developed for EVEX detection (EVEX.F04 and EVEX.R04) were added for comparison (Table 1) (van Beurden et al., 2011). Real time PCR reactions were carried out in 384-wells plates on a Light Cycler™ 480 Instruments (Roche, USA). qPCR analysis was performed with 5 μL cDNA (1/100 dilution) in a total volume of 10 μL containing the High Resolution Melting master mix (Roche, USA) (using the Resolight® set of fluorescent dyes), 3.0 mM $MgCl_2$ and 0.5 μM of each forward and reverse primer. The amplification protocol consisted of a 10 min of pre-incubation at 95ºC followed by 40 cycles using the following conditions: denaturing step at 95ºC for 15 s (ramp rate, 4.4 ºC/s), touchdown annealing from 65 to 53ºC for 15 s (ramp rate, 2.2 ºC/s), and extension at 72ºC for 20 s (ramp rate, 4.4 ºC/s). Prior to obtain HRM curves, the PCR products were incubated at 95º C for 1 min, followed by a hybridization step of 40 ºC for 1 min. Melting curves were then generated by ramping from 65 to 95ºC at 0.02ºC/s, taking 25 acquisitions per each degree centigrade. The resulting qRT-PCR products were sequenced for each couple of primers. A negative 'no-template' control (NTC) was included in the PCR to detect potential DNA contamination.

**Example 7: Real-time PCR data analyses**

For each reaction, the crossing point (Cp) (corresponding to the cycle threshold (Ct)) was determined using the "second derivative max method" applied by the LightCycler®480 Software, v.1.5.0.39 (Roche Diagnostics). The PCR efficiency (E) of each primer pair was calculated by determining the slope of standard curves obtained from serial-dilution analysis of cDNAs pooled from all experimental samples, as described by Yuan et al. (2006). The individual q-RT-PCR efficiencies (E) for the viral target or host reference genes were calculated using the formula: $E=10^{(-1/slope)}$. RNA from virus was quantified by qPCR using specific primers of 2 to 5 viral genes and calibrated using 3 references host genes. For each viral gene, the level of transcription was normalized using the mean geometric transcription rate of three reference sequences encoding ribosomal protein genes from the host, *A. anguilla*: 60S acidic ribosomal protein p0 (eel_rep_c57236, EeelBase), ribosomal protein L22 (eeel2_c1866, EeelBase) and ribosomal protein s18 (eeel2_s7245, EeelBase). The normalized relative quantities (NRQs) were calculated using the modified equation as described by Hellemans (2007):

$$NRQ = \frac{E_{t\arg et}^{\Delta Ct,t\arg et}}{\sqrt[3]{\prod_{i=1}^{3} E_{ref_i}^{\Delta Ct,ref_i}}}$$

where $E_{target}$ is the amplification efficiency of the gene of interest; $E_{ref}$ is the amplification efficiency of the reference gene; ΔCt, ref = $Ct_{ref}$(calibrator) - $Ct_{ref}$(sample); and ΔCt, target = $Ct_{target}$(calibrator) - $Ct_{target}$(sample). The calibrator corresponded to the viral RNA without host RNA. Data are then compared to standards concentrations. Regardless of selected method, at least four standard concentrations must be used for comparison of melting curve patterns when unknown samples are analyzed (*e.g.* if a specimen is screened for virus detection and quantification for comparison).

**Example 8: High Resolution Melting data analyses**

The HRM Genotyping method included in the LightCycler®480 Software, v.1.5.0.39 (Roche Diagnostics) was used to analyze the PCR products based on the melting curves as previously described by the inventors (Meistertzheim et al., 2012) and using EVA and EVEX C30 as reference. Clustering using grouping option obtained by HRM was compared with the PCR fragment sequence. Standard curves were also used as references in each PCR to standardize the calibration between samples. Each positive amplification with a peak pattern that differed from the standard curves was double-pass sequenced to verify the presence of mutations and to determine the exact nucleotide change (Genoscreen, Lille, France).

***Results***

**Example 9: Primer performance and optimization**

The specificity of the primers was analyzed by gel electrophoresis and sequencing of the qPCR products. Positives samples always generated a single peak in the melting curve analysis and a single band of the expected amplicon size in the gel electrophoresis. Negative control (NTC) did not result in a PCR product indicating that no self-primers or cross-primers were formed. Housekeeping genes (reference) were not amplified starting from viral RNA, and *vice versa* viral genes were not amplified starting from host RNA. Primers of viral and reference genes were selected to calibrate the amplification efficiency between 1.85 and 1.99 (see Table 2 hereafter).

**Example 10: Specificity**

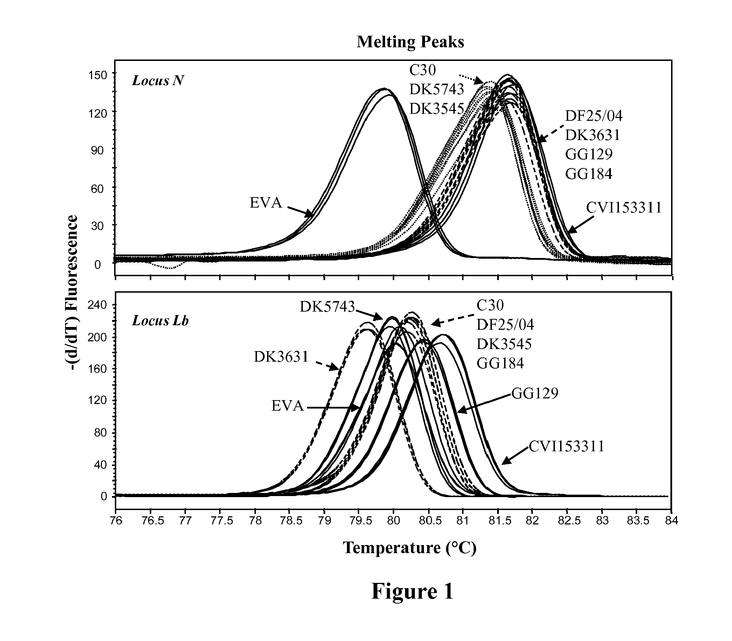

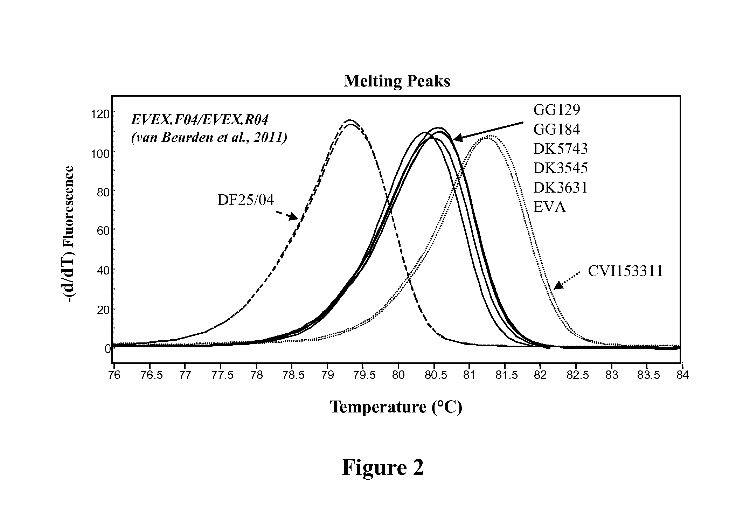

The specificity of the RT-qPCR assay was determined by testing the following strains at similar RNA concentration: the three EVEX (C30, CVI153311 and GG129) and the EVA reference strains, the three Danish EVEX isolates (DK5743, DK3545, DK3631, confirmed as positive by virological assay), one additional Dutch eel rhabdovirus (DF03/03) and eleven related fish rhabdoviruses, and non-infected yellow eels and glass eels (confirmed as negative by virological assay). Based on the virus genes, the assays readily identify and quantify EVA and EVEX strains with similar cycle threshold (Ct values). No PCR products were generated from rhabdovirus (DF03/03), the eleven related fish rhabdoviruses or the negative control. For each locus, two different melting profiles correspond to more than 2 mutations at the locus between the strains. All the loci discriminate EVA from the EVEX strains (except the P locus), with particular efficiency for the loci N (Figure 1) and M. Coupling the loci N and Lb, 77% of the different variants of EVEX and EVA were identified (Figure 1). However, the other loci allowed the differentiation of each variant with a specific melting curve at least at 1 locus and a specific HRM pattern on all the loci (Table 3). Primers previously developed for EVEX and EVA detection (van Beurden et al., 2011) didn't succeed to discriminate EVA from EVEX strains (Figure 2), but allowed the differentiation of some particular strains of EVEX (DF25/04 and CVI153311).Nonetheless, efficiencies of amplification with these last primers differ in function of the strains of EVEX indicated by the lowest coefficient of correlation obtained after amplification of RNA standard curves (EVEX and total consensus regression with $R^2$ of 0.74 and 0.09, respectively, Table 2).

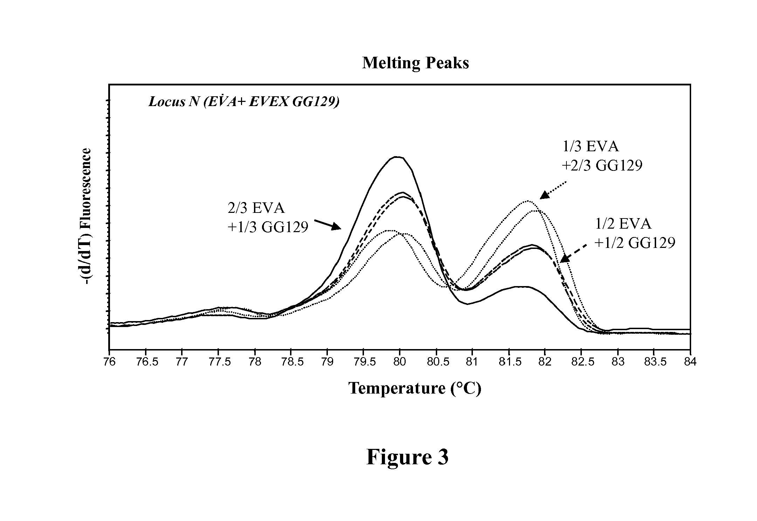

To test for detection of several viruses in one sample, viral RNA from each different strain were mixed 2 by 2 at different proportions with host RNA (total of 500 ng). EVeel-Test was applied on these artificial mixtures at the 6 loci in triplicate. The melting curves obtained revealed heteroduplexes with a detection rate of 100% (Figure 3, mixed EVA and EVEX GG129 at different proportions as an example).

**Example 11: Sensitivity**

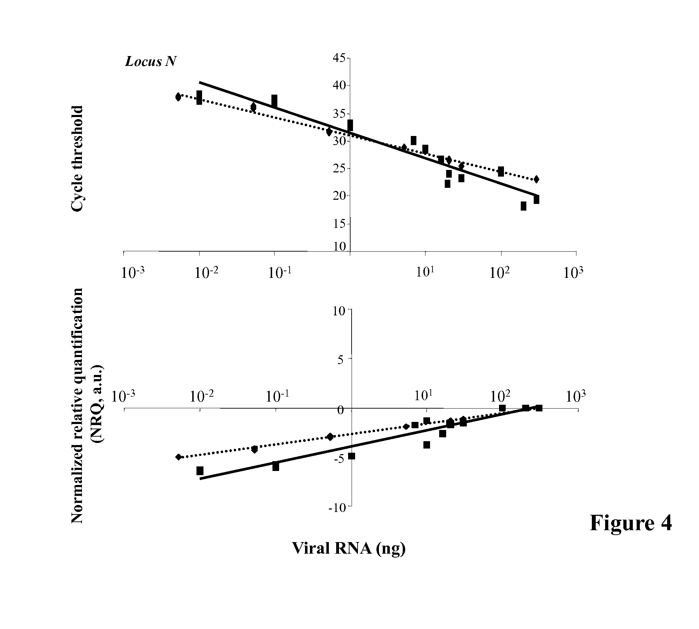

The detection limit of the RT-qPCR assay was determined by testing for the lowest detectable concentration of virus RNA mixed to host RNA in the standard curves. Linear amplification was observed between $5.10^{-3}$ to $5.10^{2}$ ng of RNA (example for locus N, Figure 4) for all strains (EVEX and EVA) corresponding to a range of viral RNA from $10^{-5}$ to 1 ng/Total $RNA_{ng}$. One molecule of total viral RNA of EVEX CVI153311 corresponding to $2.3.10^{-13}$ ng (number of each monophosphate nucleoside divided by Avogadro constant $N_A$), the low limit of our molecular test corresponds to $4.10^{9}$ molecules of viral RNA. Standardization of amplification of viral gene by host gene improved the coefficient of correlation, by taking into account the quality of total RNA (see below). The locus 18S indicated the level of total RNA and the locus L22 the percentage of degraded RNA in the sample.

**Example 12: Field samples**

Among the 60 live yellow and silver eels, collected between September 2008 and 2011 in Salses-Leucate lagoon, only two eels were detected positive by the EVeel-Test, each of the two individuals being infected by only one EVEX strain. The virological test confirmed this detection, according to ANSES. One EVEX strain (corresponding to GG184) was detected in the heart of the first a silver eel at $7.10^{-4}$ng/$TotalRNA_{ng}$, and the second EVEX strain (corresponding to GG129) was identified in the brain and spleen of another silver eel at 0.14ng and 0.09ng/$TotalRNA_{ng}$, respectively (calculated with EVEX consensus equations: $RNA_{ng}=10^{(Ct-31.38)/-4.60}$ and $RNA_{ng}=10^{(NRQ+3.94)/1.64}$). Detection of EVEX virus was also positive in 11 out of 16 pools of 10 glass eels each collected in Atlantic French estuaries, and previously detected as positive by virological assays.

**Example 13: Limits of the methods**

EVeel-Test was realized starting from 100 μL of thawed supernatant of 16 pools of 10 glass eels each after serial freezing and thawing. After only one freezing/thawing, 100% of the positive pools were detected as positive by the six viral genes, after 2, 3 and 4 freezing/thawing, only 91%, 36% and 18% were detected, respectively. Degradation of RNA was indicated by lowest Ct values of the host locus L22 (from 27 to 32).

**Example 14: Discussion**

The six loci used as target for EVeel-Test allowed strong discrimination of EVEX variants. After only 45 cycles of amplification and HRM analysis (1h15), detection, identification and quantification of virus in one host tissues is realized in few minutes with similar results compared to virological assays, performed in more than 3 days in pooled tissues. Interestingly, novel variant detect by EVeel-Test could be directly sequenced for mutations identification.

EVeel-Test allowed the identification of several viruses at different proportions in an artificial mixture as previously described for human virus (Lee et al., 2011). EVeel-Test exceeded this last study by quantify the global concentration of several viruses in the sample with high efficient PCR efficiency.

**REFERENCES**

Dixon, P.F., Hill, B.J., 1984. Rapid detection of fish rhabdoviruses by the enzyme-linked immunosorbent assay (ELISA). Aquaculture 42, 1-12.

Galinier, R., van Beurden, S., Elsa Amilhat, E., Castric, J., Schoehnh, G., Verneau, O., Fazio, G., Allienne, J.F., Engelsmac, M., Sasal, P., Faliex, E., 2012. Complete genomic sequence and taxonomic position of eel virus European X (EVEX), a rhabdovirus of European eel. Virus Research doi:10.1016/j.virusres.2012.02.020.

Hellemans, J., Mortier, G., De Paepe, A., Speleman, F., Vandesompele, J., 2007. qBase relative quantification framework and software for management and automated analysis of real-time quantitative PCR data. Genome Biology 8, R19.

Lee, H.K., Lee, C.K., Loh, T.P., Tang, J.W.-T., Tambyah, P.A., Koay, E.S.-C., 2011. High-Resolution Melting Approach to Efficient Identification and Quantification of H275Y Mutant Influenza H1 N1/2009 Virus in Mixed-Virus-Population Samples. Journal of Clinical Microbiology 49, 3555-3559.

Liew, M., Seipp, M., Durtschi, J., Margraf, R.L., Dames, S., Erali, M., Voelkerding, K., Wittwer, C., 2007. Closed-Tube SNP Genotyping Without Labeled Probes. American Journal of Clinical Pathology 127, 341-348.

Meistertzheim, A.L., Calves, I., Artigaud, S., Friedman, C.S., Laroche, J., Paillard, C., Ferec, C., 2012. High Resolution Melting Analysis for fast and cheap polymorphism screening of marine populations. Protocol Exchange.

Sano, T., 1976. Viral diseases of cultured fishes in Japan. Journal of Fish Pathology 10, 221-226.

Sano, T., Nishimura, T., Okamoto, N., Fukuda, H., 1976. Isolation of rhabdovirus from European eels (Anguilla anguilla) at Japanese port of entry. Fish Health News 5, 5-6.

van Beurden, S.J., Voorbergen-Laarman, M.A., Roozenburg, I., Boerlage, A.S., Haenen, O.L.M., Engelsma, M.Y., 2011. Development and validation of a two-step real-time RT-PCR for the detection of eel virus European X in European eel, Anguilla anguilla. Journal of Virological Methods 171, 352-359.

Yuan, J.S., Reed, A., Chen, F., Stewart, C.N., Jr., 2006. Statistical analysis of real-time PCR data. BMC Bioinformatics 7, 85-97.

## Claims

1. A method for detecting, identifying and/or quantifying a pathogen in a tissue sample of an individual, and/or for diagnosing an infection by a pathogen in a tissue sample of an individual, said method comprising the steps of:

   (a) extracting the RNA from cells of said tissue sample;
   (b) producing complementary DNA (cDNA) from the RNA extracted at step (a) by reverse transcription;
   (c) amplifying the cDNA produced at step (b) by quantitative real time polymerase chain reaction (qRT-PCR);
   (d) performing a High Resolution Melt (HRM) analysis of the qRT-PCR products obtained at step (c);
   (e) obtaining a Cycle threshold (Ct) or a Crossing point (Cp) from the qRT-PCR reaction of step (c);
   (f) obtaining a melting curve from the HRM analysis results obtained at step (d);
   (g) comparing the melting curve obtained at step (f) to a pathogen reference melting curve and/or comparing the Ct or Cp obtained at step (e) to a pathogen or host reference standard curve;
   (h) deducing the presence, the identity and/or the quantity of said pathogen in said tissue sample of the individual, and/or deducing that the individual is infected by said pathogen, if the curve obtained at step (f) is similar to said reference curve, and/or the Ct or Cp obtained at step (e) is similar to said reference standard curve.

2. A method according to claim 1 for detecting and/or identifying a pathogen in a tissue sample of an individual, said method comprising the steps of:

   (a) extracting the RNA from cells of said tissue sample;
   (b) producing complementary DNA (cDNA) from the RNA extracted at step (a) by reverse transcription;
   (c) amplifying the cDNA produced at step (b) by quantitative real time polymerase chain reaction (qRT-PCR);
   (d) performing a High Resolution Melt (HRM) analysis of the qRT-PCR products obtained at step (c);
   (e) obtaining a melting curve from the HRM analysis results obtained at step (d);
   (f) comparing the melting curve obtained at step (e) to a pathogen reference melting curve;
   (g) deducing the presence and/or the identity of said pathogen in said tissue sample of the individual, and/or deducing that the individual is infected by said pathogen, if the curve obtained at step (e) is similar to the reference curve.

3. A method according to claim 1 for quantifying a pathogen in a tissue sample of an individual, said method comprising the steps of:

   (a) extracting the RNA from cells of said tissue sample;
   (b) producing complementary DNA (cDNA) from the RNA extracted at step (a) by reverse transcription;
   (c) amplifying the cDNA produced at step (b) by quantitative real time polymerase chain reaction (qRT-PCR);

(d) performing a High Resolution Melt (HRM) analysis of the qRT-PCR products obtained at step (c);
(e) obtaining a Cycle threshold (Ct) or a Crossing point (Cp) from the qRT-PCR reaction of step (c);
(f) obtaining a melting curve from the HRM analysis results obtained at step (d);
(g) comparing the Ct or Cp obtained at step (e) to a pathogen or host reference standard curve;
(h) deducing the quantity of said pathogen in said tissue sample of the individual from the comparison of step (g).

**4.** A method according to claim 1 for diagnosing an infection by a pathogen in a tissue sample of an individual, said method comprising the steps of:

(a) extracting the RNA from the cells of said tissue sample;
(b) producing complementary DNA (cDNA) from the RNA extracted at step (a) by reverse transcription;
(c) amplifying the cDNA produced at step (b) by quantitative real time polymerase chain reaction (qRT-PCR) using primers;
(d) performing a High Resolution Melt (HRM) analysis of the qRT-PCR products obtained at step (c);
(e) obtaining a melting curve from the HRM analysis results obtained at step (d);
(f) comparing the melting curve obtained at step (e) to a reference melting curve of said pathogen;
(g) deducing that the individual is infected by said pathogen if the curve obtained at step (e) is similar to the reference curve.

**5.** The method of any one of claims 1 to 4, wherein said pathogen is a virus, a bacterium, a fungus, a prion, a protist or a helminth producing RNA.

**6.** The method of any one of claims 1 to 5, wherein said pathogen is a virus.

**7.** The method of any one of claims 1 to 6, wherein said pathogen is a rhabdovirus.

**8.** The method of any one of claims 1 to 7, wherein said pathogen is EVEX or EVA.

**9.** The method of any one of claims 1 to 8, wherein said individual is an animal.

**10.** The method of any one of claims 1 to 9, wherein said individual is an eel.

**11.** The method of any one of claims 1 to 10, wherein the quantitative real time polymerase chain reaction (qRT-PCR) is performed using:

- at least one primer having a sequence at least 80% identical to a sequence selected from the group consisting of SEQ ID NO: 1 to 18; or
at least one primer capable of hybridizing to a sequence selected from the group consisting of SEQ ID NO: 25 to 36, or sequences complementary thereto; or
- at least one primer capable of amplifying a sequence at least 80% identical to a sequence selected from the group consisting of SEQ ID NO: 19-24.

**12.** The method of any one of claims 1 to 11, wherein the high resolution melt (HRM) analysis is performed using at least one dye selected from the group consisting of ResoLight,and Chromofy.

**13.** The method of any one of claims 1 to 12, wherein the reference melting curve or the pathogen or host reference standard curve is obtained from the EVA "Hill" J6BF4 (GenBank accession number: KC608038), the French EVEX C30 (accession number: JN639009), GG129 (accession number: JN639010) or GG184 (accession number: KC608033), the Dutch EVEX CVI153311 (accession number: FN557213), the German EVEX DF25/04 (accession number: KC608037) or the Danish EVEX DK3545, DK3631 or DK5743 (accession numbers: KC608034, KC608035 and KC608036 respectively).

**14.** A kit comprising:

- at least one primer having a sequence at least 80% identical to a sequence selected in the group consisting of SEQ ID NO: 1 to 18; and/or
- optionally, at least one reference melting curve obtained from the EVA "Hill" J6BF4 (GenBank accession number: KC608038), the French EVEX C30 (accession number: JN639009), GG129 (accession number:

JN639010) or GG184 (accession number: KC608033), the Dutch EVEX CVI153311 (accession number: FN557213), the German EVEX DF25/04 (accession number: KC608037) or the Danish EVEX DK3545, DK3631 or DK5743 (accession numbers: KC608034, KC608035 and KC608036 respectively);
- optionally, at least one control sample of known concentration of nucleic acid from EVA "Hill" J6BF4 (GenBank accession number: KC608038), French EVEX C30 (accession number: JN639009), GG129 (accession number: JN639010) or GG184 (accession number: KC608033), Dutch EVEX CVI153311 (accession number: FN557213), German EVEX DF25/04 (accession number: KC608037), or the Danish EVEX DK3545, DK3631 or DK5743 (accession numbers: KC608034, KC608035 and KC608036 respectively);
- optionally, at least one control sample of known concentration of nucleic acid of reference genes from a host; and
- optionally, instructions for use.

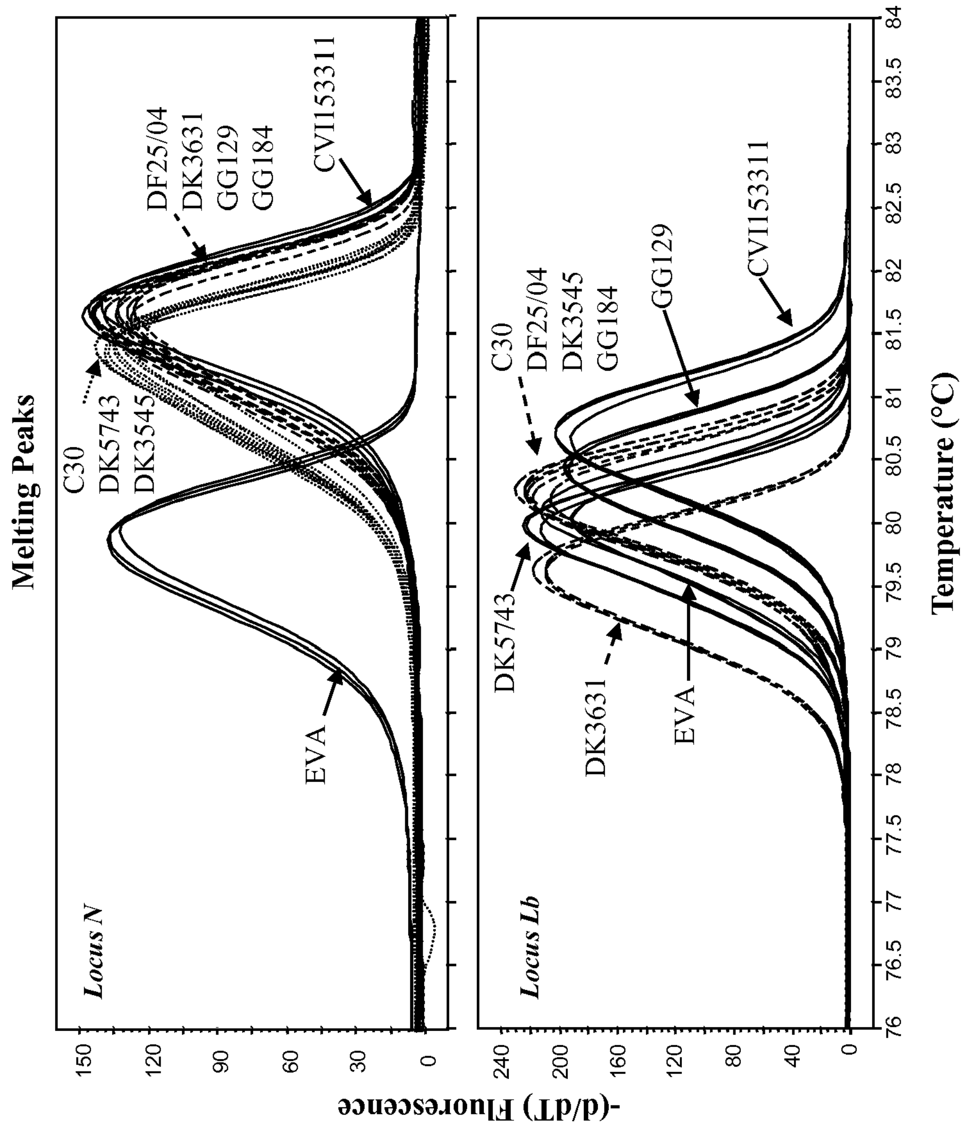

Melting Peaks
Locus N
EVA
C30
DK5743
DK3545
DF25/04
DK3631
GG129
GG184
CVI153311
Locus Lb
DK3631
EVA
DK5743
C30
DF25/04
DK3545
GG184
GG129
CVI153311
-(d/dT) Fluorescence
Temperature (°C)


**Figure 1**

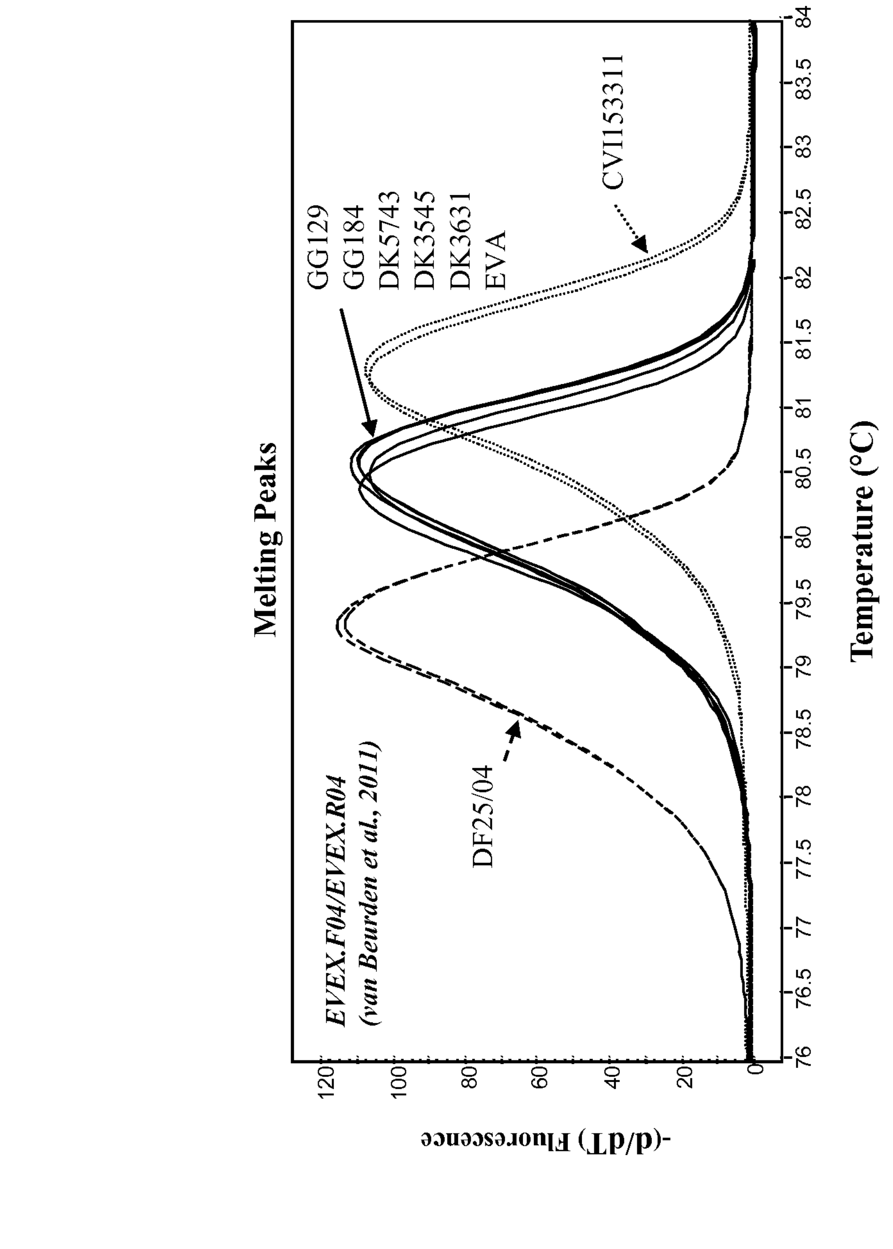
Melting Peaks
EVEX.F04/EVEX.R04
(van Beurden et al., 2011)
DF25/04
GG129
GG184
DK5743
DK3545
DK3631
EVA
CVI153311
-(d/dT) Fluorescence
0
20
40
60
80
100
120
76
76.5
77
77.5
78
78.5
79
79.5
80
80.5
81
81.5
82
82.5
83
83.5
84
Temperature (°C)

Figure 2

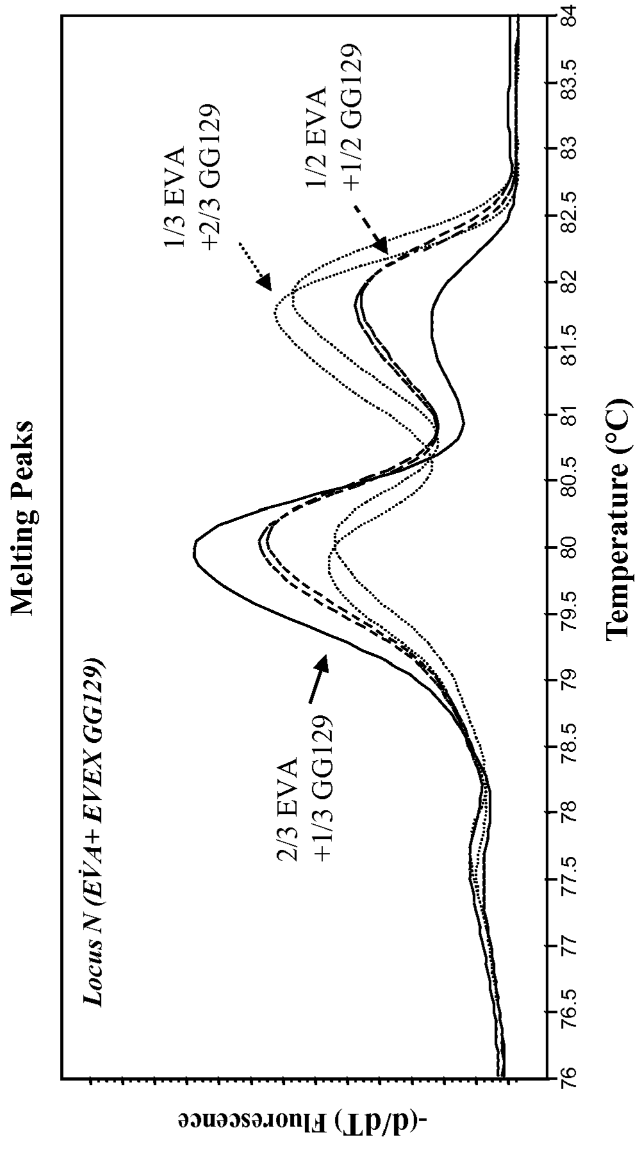
Melting Peaks
Locus N (EVA+ EVEX GG129)
2/3 EVA
+1/3 GG129
1/3 EVA
+2/3 GG129
1/2 EVA
+1/2 GG129
-(d/dT) Fluorescence
Temperature (°C)
76
76.5
77
77.5
78
78.5
79
79.5
80
80.5
81
81.5
82
82.5
83
83.5
84

Figure 3

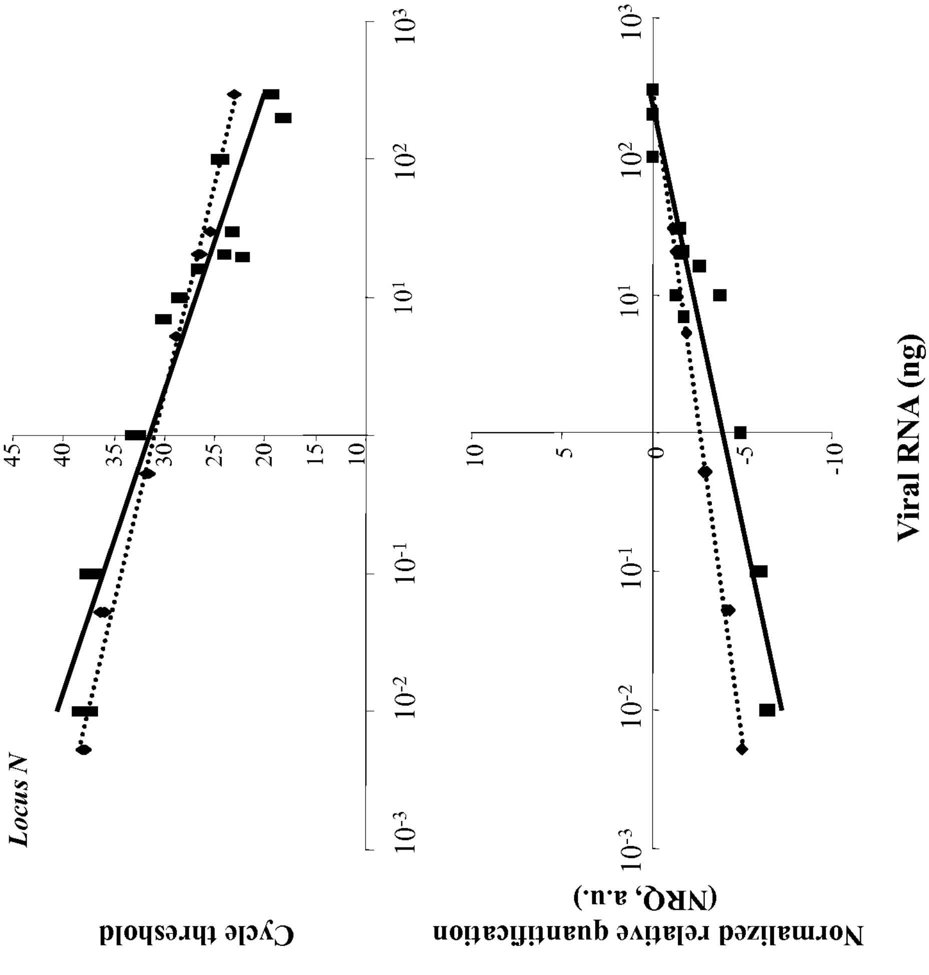

Locus N
Cycle threshold
45
40
35
30
25
20
15
10
Normalized relative quantification
(NRQ, a.u.)
10
5
0
-5
-10
$10^{-3}$
$10^{-2}$
$10^{-1}$
$10^{1}$
$10^{2}$
$10^{3}$
Viral RNA (ng)


**Figure 4**

# Sequence of the N locus

```
                         10        20        30        40        50        60        70        80        90       100
                ....|....|....|....|....|....|....|....|....|....|....|....|....|....|....|....|....|....|....|....|
EVEX_CVI153311  GAAGGCACTGAAGAATGTCACTACTCTAATCAACAGTGCCAACTATCTGAGAATGGTGGCAGTTATTGACATGTTCTACTACCACTTCAAGAACAGTCAA
EVEX_GG129      ...........................C...................................G....................T...............
EVEX_C30        ...........................C......................A............G....................T...............
EVEX_GG184      ...........................C...................................G....................T...............
EVEX_DF25/04    ...........................C...................................G....................T...............
EVEX_DK3545     ...........................C......................A............G....................T...............
EVEX_DK3631     ...........................C......................A............G....................T...............
EVEX_DK5743     ...........................C......................A............G....................T...............
EVA             ...........................T..T........A.......................G.....T..............T...............

                        110       120       130       140
                ....|....|....|....|....|....|....|....|
EVEX_CVI153311  GACAGAGCTGTTGTCAGGATTGCCACTCTGAGTTCCCGTC
EVEX_GG129      ..A.....................................
EVEX_C30        ..G.....................................
EVEX_GG184      ..A.....................................
EVEX_DF25/04    ..A.....................................
EVEX_DK3545     ..A.....C.............................C.
EVEX_DK3631     ..A.....................................
EVEX_DK5743     ..A.....................................
EVA             ..A..............A......................
```

**Figure 5**

# Sequence of the M locus

```
                         10        20        30        40        50        60        70        80        90       100
                ....|....|....|....|....|....|....|....|....|....|....|....|....|....|....|....|....|....|....|....|
EVEX_CVI153311  GGCCCGATCCTTACCCGTCCGTTTTACATTGCCTTGTTCTTGGGCGGGATCCACGGGATGCAAGCGGGAGTTAAAGGAGCGAGGAGCATCCGGTATGAAA
EVEX_GG129      ....................A.........................................G....................A................
EVEX_C30        ...................................................................................A................
EVEX_GG184      ..............................................................T......T.............A................
EVEX_DF25/04    ....................A.........................................G....................A................
EVEX_DK3545     ..............................................................G.....G..............A................
EVEX_DK3631     ...........G......................................T...........G.....G..............A................
EVEX_DK5743     ...................................................................................A.......T........
EVA             ....................T........A........T...........A...........G..A.....C...........A......A.......G.

                        110       120       130       140
                ....|....|....|....|....|....|....|....|....|..
EVEX_CVI153311  GAGAACATCATGGACCTCTGGTGTTCCCATATCACAGAAGCAACCCG
EVEX_GG129      ...............................................
EVEX_C30        ...............................................
EVEX_GG184      ...............................................
EVEX_DF25/04    ...............................................
EVEX_DK3545     ...............................................
EVEX_DK3631     ...............................................
EVEX DK5743     ...............................................
EVA             ..........G....................................
```

**Figure 6**

# Sequence of the G locus

```
                        10        20        30        40        50        60        70        80        90       100
               ....|....|....|....|....|....|....|....|....|....|....|....|....|....|....|....|....|....|....|....|
EVEXCVI153311  ACGGAGAGTGTGACATACACAACTGTATCATCTCACGAGGTAAAATTAGATCCATATCAGATGACTTTCGTGGATTCACTCTTTCCGGGCGGTAAATGCT
EVEX_GG129     ........................................................C.............C.............................
EVEX_C30       ........................................................C...........................................
EVEXGG184      ........................................................C...........................................
EVEX_DF25/04   ........................................................C.......................T..C................
EVEXDK3545     .............................................C..........C................................T..........
EVEXDK3631     ........................................................C...........................................
EVEXDK5743     ..................................C.....................C...........................................
EVA            .............................................C..........C...........................................

                       110       120       130       140       150       160       170       180       190       200
               ....|....|....|....|....|....|....|....|....|....|....|....|....|....|....|....|....|....|....|....|
EVEXCVI153311  CTTCTTCAGTGTGTAGCACCATTTATCATCAAGGAGTCTGGATAAACCCGAGTAACAACTTAGGGTTTTGCAAAGATCCGGTCGATCATCAAGGTCAGTT
EVEX_GG129     .C...............................................T........T.....A...................................
EVEX_C30       .C..................................................................................................
EVEXGG184      .C...............................................T........T.....A...................................
EVEX_DF25/04   .C...........................................G.......G.........................A....................
EVEXDK3545     .C.....G..............C...............................C.........................A....................
EVEXDK3631     .C.....G..............C...............................C.........................A....................
EVEXDK5743     .C..................................................C..T............................................
EVA            .C.............A...T..C.........................T..A.G..................G...A..A....................

                       210
               ....|....|....|
EVEXCVI153311  GTACATGGCTGGTCT
EVEX_GG129     ...............
EVEX_C30       ...............
EVEXGG184      ...............
EVEX_DF25/04   ...............
EVEXDK3545     ...............
EVEXDK3631     ...............
EVEXDK5743     ...............
EVA            ...............
```

Figure 7

# Sequence of the P locus

```
                          10        20        30        40        50        60        70        80        90       100
                 ....|....|....|....|....|....|....|....|....|....|....|....|....|....|....|....|....|....|....|....|
EVEX_CVI153311   CCACAGATGAGAAGCCTGAACATCTCTCTGATGCATTCTCAAAATACACAGAATATTTGTCCAATGAGAGTAAGGAGGAGGAGGAGGATTTCCAATTTGA
EVEX_GG129       ...............................C.............................A........C....G...T....................
EVEX_C30         ...............................C......................................C....G.....................C..
EVEX_GG184       ...............................C.............................A........C....G...T....................
EVEX_DF25/04     ...............................C......................................C....G...A..A..............C..
EVEX_DK3545      ............................G..C.....T.......................A........C....T.....................C..
EVEX_DK3631      ...............................C.....T.......................A........C....G.....................C..
EVEX_DK5743      ...............................C......................................C....G.....................C..
EVA              ...............................C...........G..T.........C.............C....T...............G.....C..

                         110       120       130       140       150       160
                 ....|....|....|....|....|....|....|....|....|....|....|....|.
EVEX_CVI153311   ACAGGTAGACTATGGATTTCAAGAGTCACCTGAATCCAACAACCCTCTGCAAGAGAGCTGG
EVEX_GG129       ......G................................T.....................
EVEX_C30         ......G................................T.....................
EVEX_GG184       ......G................................T.....................
EVEX_DF25/04     ......G................................T.....................
EVEX_DK3545      ......G..A..............A..............T.....................
EVEX_DK3631      ......G..A.............................T.....................
EVEX_DK5743      ......G................................T.....................
EVA              ......G.................A.....G..G.....T.....................
```

**Figure 8**

# Sequence of the La locus

```
                        10        20        30        40        50        60        70        80        90       100
                ....|....|....|....|....|....|....|....|....|....|....|....|....|....|....|....|....|....|....|....|
EVEX_CVI153311  GGCATCACAGAATCTCTCATTTCTTTATTCCAAAATTCCAAAACCATTCGCAACATCTTCAGGAAGAAATATGCGAAAGAATTGGAACTTAGAGTAGTGC
EVEX_GG129      ........................................................A...........................................
EVEX_C30        ....................................................................................................
EVEX_GG184      ........................................................A...........................................
EVEX_DF25/04    ..........................................................................A.........................
EVEX_DK3545     ..........................................................................A.........................
EVEX_DK3631     ....................................................................................................
EVEX_DK5743     ....................................................................................................
EVA             ......................................T....................T..A...........A........A................

                       110       120       130       140       150       160       170       180       190       200
                ....|....|....|....|....|....|....|....|....|....|....|....|....|....|....|....|....|....|....|....|
EVEX_CVI153311  AGTGTGAATACAGATCCATCAATTTGATGTTGAGCCTCGCAGATCGATCCCACTTAGATGAAATGTGGACATGCTCGGCATCTAGAGCAGACGAACTCCG
EVEX_GG129      .............................C...............A......................................A...............
EVEX_C30        ....................................................................................A...............
EVEX_GG184      .............................C...............A......................................A...............
EVEX_DF25/04    .A..................................................................................A...G........T..
EVEX_DK3545     ....................................................T...............................A......T........
EVEX_DK3631     ....................................................................................A......T........
EVEX_DK5743     ....................................................................................A...............
EVA             .......G...............C..........T..................C.G.................T..A.....C.A...G..T........

                       210       220       230
                ....|....|....|....|....|....|....|..
EVEX_CVI153311  AACCTTATCATGGGGGACTACAATAATTGGAACAACT
EVEX_GG129      .....................................
EVEX_C30        .....................................
EVEX_GG184      .....................................
EVEX_DF25/04    ....C................................
EVEX_DK3545     .....................................
EVEX_DK3631     .....................................
EVEX_DK5743     .....................................
EVA             ...AC....T...........................
```

**Figure 9**

# Sequence of the Lb locus

```
                        10        20        30        40        50        60        70        80        90       100
                ....|....|....|....|....|....|....|....|....|....|....|....|....|....|....|....|....|....|....|....|
EVEX_CVI153311  GGAATTGGGACAGATTGACACATCAGGAGCAAAGTTTCCAGGTGGGGAAGTCCATCGGGTTTTTGTTTGGAGATTTGACAATGACAAAAAATTCACATGC
EVEX_GG129      .....................................T...A.A..................C.........................G...........
EVEX_C30        .....................................T...A.A..................C.........................G...........
EVEX_GG184      .....................................T...A.A..................C.........................G...........
EVEX_DF25/04    .....................................T...A.A........T.........C.........................G...........
EVEX_DK3545     .....................................T...A.A..................C.........................G...........
EVEX_DK3631     ....C.......................A........T...A.A........T.........C.........................G...........
EVEX_DK5743     .....................................T...A.A...........T......C.........................G...........
EVA             ............................A..G.....T...A.A.....A..T.........C.A...........A........C..G...........

                       110       120       130       140       150       160       170       180       190
                ....|....|....|....|....|....|....|....|....|....|....|....|....|....|....|....|....|....|..
EVEX_CVI153311  TCAGGATTCCTCTATTTTCCCTTTATCAATTCAATACAAGATCACAGCTGCGGAGTTTCTGGAAGGAATTCTAGATGGGATAGTCAAGGCTT
EVEX_GG129      C........T..................................................................................
EVEX_C30        .........T..................................................................................
EVEX_GG184      C........T..................................................................................
EVEX_DF25/04    .........T..............G...................................................................
EVEX_DK3545     .........T..................................................................................
EVEX_DK3631     .........T..................................................................................
EVEX_DK5743     .........T..................................................................................
EVA             ...A..C..T...........AC.G.......................C........C..A...............................
```

**Figure 10**

European Patent Office

# EUROPEAN SEARCH REPORT

Application Number
EP 13 30 5937

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VAN BEURDEN S J ET AL: "Development and validation of a two-step real-time RT-PCR for the detection of eel virus European X in European eel, Anguilla anguilla", JOURNAL OF VIROLOGICAL METHODS, ELSEVIER BV, NL, vol. 171, no. 2, 1 February 2011 (2011-02-01), pages 352-359, XP027595362, ISSN: 0166-0934, DOI: 10.1016/J.JVIROMET.2010.11.019 [retrieved on 2011-01-11] * the whole document * | 1,3, 5-11,13 | INV. C12Q1/70 |
| X | H. K. LEE ET AL: "High-Resolution Melting Approach to Efficient Identification and Quantification of H275Y Mutant Influenza H1N1/2009 Virus in Mixed-Virus-Population Samples", JOURNAL OF CLINICAL MICROBIOLOGY, vol. 49, no. 10, 24 August 2011 (2011-08-24), pages 3555-3559, XP055088779, ISSN: 0095-1137, DOI: 10.1128/JCM.01087-11 * the whole document * | 1,2,4-6 | TECHNICAL FIELDS SEARCHED (IPC): C12Q |
| A | S P JONSTRUP ET AL: "Development and validation of a novel Taqman-based real-time RT-PCR assay suitable for demonstrating freedom from viral haemorrhagic septicaemia virus", JOURNAL OF FISH DISEASES, vol. 36, no. 1, 27 January 2013 (2013-01-27), pages 9-23, XP055088668, ISSN: 0140-7775, DOI: 10.1111/j.1365-2761.2012.01416.x | 1-14 | |

-/--

The present search report has been drawn up for all claims

1

| Place of search | Date of completion of the search | Examiner |
|---|---|---|
| The Hague | 19 November 2013 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 30 5937

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 102 676 641 A (SHANGHAI YIRUN BIOLOG TECHNOLOGY CO LTD) 19 September 2012 (2012-09-19) * sequence 7 * & DATABASE Geneseq [Online] 23 May 2013 (2013-05-23), "Human 18S rRNA gene-specific foward PCR primer, SEQ: 7.", retrieved from EBI accession no. GSN:BAM84472 Database accession no. BAM84472 * sequence * ----- | 14 | |

TECHNICAL FIELDS SEARCHED (IPC)

The present search report has been drawn up for all claims

1

| Place of search | Date of completion of the search | Examiner |
|---|---|---|
| The Hague | 19 November 2013 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 30 5937

This annex lists the patent family members relating to the patent documents cited in the above-mentioned European search report. The members are as contained in the European Patent Office EDP file on
The European Patent Office is in no way liable for these particulars which are merely given for the purpose of information.

19-11-2013

| Patent document cited in search report | Publication date | Patent family member(s) | Publication date |
|---|---|---|---|
| CN 102676641 A | 19-09-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAMBROOK et al.** Molecular Cloning: A Laboratory Manual. Cold Spring Harbor Press, 1989
- **DIXON, P.F. ; HILL, B.J.** Rapid detection of fish rhabdoviruses by the enzyme-linked immunosorbent assay (ELISA. *Aquaculture,* 1984, vol. 42, 1-12
- **GALINIER, R. ; VAN BEURDEN, S. ; ELSA AMILHAT, E. ; CASTRIC, J. ; SCHOEHNH, G. ; VERNEAU, O. ; FAZIO, G. ; ALLIENNE, J.F. ; ENGELSMAC, M. ; SASAL, P.** Complete genomic sequence and taxonomic position of eel virus European X (EVEX), a rhabdovirus of European eel. *Virus Research,* 2012
- **HELLEMANS, J. ; MORTIER, G. ; DE PAEPE, A. ; SPELEMAN, F. ; VANDESOMPELE, J.** qBase relative quantification framework and software for management and automated analysis of real-time quantitative PCR data. *Genome Biology,* 2007, vol. 8, R19
- **LEE, H.K. ; LEE, C.K. ; LOH, T.P. ; TANG, J.W.-T. ; TAMBYAH, P.A. ; KOAY, E.S.-C.** High-Resolution Melting Approach to Efficient Identification and Quantification of H275Y Mutant Influenza H1 N1/2009 Virus in Mixed-Virus-Population Samples. *Journal of Clinical Microbiology,* 2011, vol. 49, 3555-3559
- **LIEW, M. ; SEIPP, M. ; DURTSCHI, J. ; MARGRAF, R.L. ; DAMES, S. ; ERALI, M. ; VOELKERDING, K. ; WITTWER, C.** Closed-Tube SNP Genotyping Without Labeled Probes. *American Journal of Clinical Pathology,* 2007, vol. 127, 341-348
- **MEISTERTZHEIM, A.L. ; CALVES, I. ; ARTIGAUD, S. ; FRIEDMAN, C.S. ; LAROCHE, J. ; PAILLARD, C. ; FEREC, C.** High Resolution Melting Analysis for fast and cheap polymorphism screening of marine populations. *Protocol Exchange,* 2012
- **SANO, T.** Viral diseases of cultured fishes in Japan. *Journal of Fish Pathology,* 1976, vol. 10, 221-226
- **SANO, T. ; NISHIMURA, T. ; OKAMOTO, N. ; FUKUDA, H.** Isolation of rhabdovirus from European eels (Anguilla anguilla) at Japanese port of entry. *Fish Health News,* 1976, vol. 5, 5-6
- **VAN BEURDEN, S.J. ; VOORBERGEN-LAARMAN, M.A. ; ROOZENBURG, I. ; BOERLAGE, A.S. ; HAENEN, O.L.M. ; ENGELSMA, M.Y.** Development and validation of a two-step real-time RT-PCR for the detection of eel virus European X in European eel, Anguilla anguilla. *Journal of Virological Methods,* 2011, vol. 171, 352-359
- **YUAN, J.S. ; REED, A. ; CHEN, F. ; STEWART, C.N., JR.** Statistical analysis of real-time PCR data. *BMC Bioinformatics,* 2006, vol. 7, 85-97

SEQUENCE LISTING

<110> CENTRE NATIONAL DE LA RECHERCHE SCIENTIFIQUE (C.N.R.S)
UNIVERSITE DE PERPIGNAN VIA DOMITIA

<120> A METHOD FOR DETECTING, IDENTIFYING AND/OR QUANTIFYING A PATHOGEN IN AN INDIVIDUAL

<130> BET 13P0999

<160> 84

<170> PatentIn version 3.4

<210> 1
<211> 26
<212> DNA
<213> Artificial

<220>
<223> primer

<400> 1
gaaggcactg aagaatgtca ctactc 26

<210> 2
<211> 17
<212> DNA
<213> Artificial

<220>
<223> primer

<400> 2
ggcccgatcc ttacccg 17

<210> 3
<211> 26
<212> DNA
<213> Artificial

<220>
<223> primer

<400> 3
acggagagtg tgacatacac aactgt 26

<210> 4
<211> 23
<212> DNA
<213> Artificial

<220>
<223> primer

<400> 4
ccacagatga gaagcctgaa cat 23

<210> 5

<211> 27
<212> DNA
<213> Artificial

<220>
<223> primer

<400> 5
ggcatcacag aatctctcat ttcttta 27

<210> 6
<211> 23
<212> DNA
<213> Artificial

<220>
<223> primer

<400> 6
ggaattggga cagattgaca cat 23

<210> 7
<211> 22
<212> DNA
<213> Artificial

<220>
<223> primer

<400> 7
atggaggcaa gagttgggta tc 22

<210> 8
<211> 17
<212> DNA
<213> Artificial

<220>
<223> primer

<400> 8
tggcagggct ggaaacc 17

<210> 9
<211> 21
<212> DNA
<213> Artificial

<220>
<223> primer

<400> 9
ataccgcagc taggaataat g 21

<210> 10
<211> 20
<212> DNA
<213> Artificial

<220>
<223>  primer

<400>  10
gacgggaact cagagtggca                                                    20

<210>  11
<211>  21
<212>  DNA
<213>  Artificial

<220>
<223>  primer

<400>  11
cgggttgctt ctgtgatatg g                                                  21

<210>  12
<211>  23
<212>  DNA
<213>  Artificial

<220>
<223>  primer

<400>  12
agaccagcca tgtacaactg acc                                                23

<210>  13
<211>  21
<212>  DNA
<213>  Artificial

<220>
<223>  primer

<400>  13
ccagctctct tgcagagggt t                                                  21

<210>  14
<211>  26
<212>  DNA
<213>  Artificial

<220>
<223>  primer

<400>  14
cagttgttcc aattattgta gtcccc                                             26

<210>  15
<211>  27
<212>  DNA
<213>  Artificial

<220>
<223>  primer

<400> 15
aagccttgac tatcccatct agaattc 27

<210> 16
<211> 21
<212> DNA
<213> Artificial

<220>
<223> primer

<400> 16
tggagggtgt gaggaatatc g 21

<210> 17
<211> 27
<212> DNA
<213> Artificial

<220>
<223> primer

<400> 17
ccagtctctc agattgttct ttttcag 27

<210> 18
<211> 18
<212> DNA
<213> Artificial

<220>
<223> primer

<400> 18
ggccgtccct cttaatca 18

<210> 19
<211> 140
<212> DNA
<213> EVEX CVI153311

<400> 19
gaaggcactg aagaatgtca ctactctaat caacagtgcc aactatctga gaatggtggc 60

agttattgac atgttctact accacttcaa gaacagtcaa gacagagctg ttgtcaggat 120

tgccactctg agttcccgtc 140

<210> 20
<211> 147
<212> DNA
<213> EVEX CVI153311

<400> 20
ggcccgatcc ttacccgtcc gttttacatt gccttgttct tgggcgggat ccacgggatg 60

caagcgggag ttaaaggagc gaggagcatc cggtatgaaa gagaacatca tggacctctg 120

gtgttcccat atcacagaag caacccg 147

<210> 21
<211> 215
<212> DNA
<213> EVEX CVI153311

<400> 21
acggagagtg tgacatacac aactgtatca tctcacgagg taaaattaga tccatatcag 60

atgactttcg tggattcact ctttccgggc ggtaaatgct cttcttcagt gtgtagcacc 120

atttatcatc aaggagtctg gataaacccg agtaacaact tagggttttg caaagatccg 180

gtcgatcatc aaggtcagtt gtacatggct ggtct 215

<210> 22
<211> 161
<212> DNA
<213> EVEX CVI153311

<400> 22
ccacagatga gaagcctgaa catctctctg atgcattctc aaaatacaca gaatatttgt 60

ccaatgagag taaggaggag gaggaggatt tccaatttga acaggtagac tatggatttc 120

aagagtcacc tgaatccaac aaccctctgc aagagagctg g 161

<210> 23
<211> 237
<212> DNA
<213> EVEX CVI153311

<400> 23
ggcatcacag aatctctcat ttctttattc caaaattcca aaaccattcg caacatcttc 60

aggaagaaat atgcgaaaga attggaactt agagtagtgc agtgtgaata cagatccatc 120

aatttgatgt tgagcctcgc agatcgatcc cacttagatg aaatgtggac atgctcggca 180

tctagagcag acgaactccg aaccttatca tgggggacta caataattgg aacaact 237

<210> 24
<211> 192
<212> DNA
<213> EVEX CVI153311

<400> 24
ggaattggga cagattgaca catcaggagc aaagtttcca ggtggggaag tccatcgggt 60

ttttgtttgg agatttgaca atgacaaaaa attcacatgc tcaggattcc tctattttcc 120

ctttatcaat tcaatacaag atcacagctg cggagtttct ggaaggaatt ctagatggga 180

tagtcaaggc tt 192

<210> 25

<211> 26
<212> DNA
<213> EVEX CVI153311

<400> 25
gaaggcactg aagaatgtca ctactc 26

<210> 26
<211> 20
<212> DNA
<213> EVEX CVI153311

<400> 26
gacgggaact cagagtggca 20

<210> 27
<211> 17
<212> DNA
<213> EVEX CVI153311

<400> 27
ggcccgatcc ttacccg 17

<210> 28
<211> 21
<212> DNA
<213> EVEX CVI153311

<400> 28
cgggttgctt ctgtgatatg g 21

<210> 29
<211> 26
<212> DNA
<213> EVEX CVI153311

<400> 29
acggagagtg tgacatacac aactgt 26

<210> 30
<211> 23
<212> DNA
<213> EVEX CVI153311

<400> 30
agaccagcca tgtacaactg acc 23

<210> 31
<211> 16
<212> DNA
<213> EVEX CVI153311

<400> 31
ccacagatga gaagcc 16

<210> 32

<211>  21
<212>  DNA
<213>  EVEX CVI153311

<400>  32
ccagctctct tgcagagggt t                                                21

<210>  33
<211>  27
<212>  DNA
<213>  EVEX CVI153311

<400>  33
ggcatcacag aatctctcat ttcttta                                          27

<210>  34
<211>  26
<212>  DNA
<213>  EVEX CVI153311

<400>  34
agttgttcca attattgtag tccccc                                           26

<210>  35
<211>  23
<212>  DNA
<213>  EVEX CVI153311

<400>  35
ggaattggga cagattgaca cat                                              23

<210>  36
<211>  27
<212>  DNA
<213>  EVEX CVI153311

<400>  36
aagccttgac tatcccatct agaattc                                          27

<210>  37
<211>  140
<212>  DNA
<213>  EVEX GG129

<400>  37
gaaggcactg aagaatgtca ctactctcat caacagtgcc aactatctga gaatggtggc      60

agtgattgac atgttctact accatttcaa gaacagtcaa gaaagagctg ttgtcaggat     120

tgccactctg agttcccgtc                                                 140

<210>  38
<211>  147
<212>  DNA
<213>  EVEX GG129

<400>  38

ggcccgatcc ttacccgtcc attttacatt gccttgttct tgggcgggat ccacgggatg 60

caggcgggag ttaaaggagc gagaagcatc cggtatgaaa gagaacatca tggacctctg 120

gtgttcccat atcacagaag caacccg 147

<210> 39
<211> 215
<212> DNA
<213> EVEX GG129

<400> 39
acggagagtg tgacatacac aactgtatca tctcacgagg taaaattaga tccataccag 60

atgactttcg cggattcact ctttccgggc ggtaaatgct cctcttcagt gtgtagcacc 120

atttatcatc aaggagtctg gataaaccct agtaacaatt taggattttg caaagatccg 180

gtcgatcatc aaggtcagtt gtacatggct ggtct 215

<210> 40
<211> 161
<212> DNA
<213> EVEX GG129

<400> 40
ccacagatga gaagcctgaa catctctctg acgcattctc aaaatacaca gaatatttgt 60

caaatgagag caaggggat gaggaggatt tccaatttga acaggtggac tatggatttc 120

aagagtcacc tgaatccaat aaccctctgc aagagagctg g 161

<210> 41
<211> 237
<212> DNA
<213> EVEX GG129

<400> 41
ggcatcacag aatctctcat ttctttattc caaaattcca aaaccattcg caacatattc 60

aggaagaaat atgcgaaaga attggaactt agagtagtgc agtgtgaata cagatccatc 120

aatttgatgc tgagcctcgc agatcaatcc cacttagatg aaatgtggac atgctcggca 180

tctaaagcag acgaactccg aaccttatca tgggggacta caataattgg aacaact 237

<210> 42
<211> 192
<212> DNA
<213> EVEX GG129

<400> 42
ggaattggga cagattgaca catcaggagc aaagttttca gatagggaag tccatcgggt 60

ttctgtttgg agatttgaca atgacaaaga attcacatgc ccaggattct tctattttcc 120

ctttatcaat tcaatacaag atcacagctg cggagtttct ggaaggaatt ctagatggga 180

tagtcaaggc tt 192

<210> 43
<211> 140
<212> DNA
<213> EVEX C30

<400> 43
gaaggcactg aagaatgtca ctactctcat caacagtgcc aactatctga aaatggtggc 60

agtgattgac atgttctact accatttcaa gaacagtcaa gagagagctg ttgtcaggat 120

tgccactctg agttcccgtc 140

<210> 44
<211> 147
<212> DNA
<213> EVEX C30

<400> 44
ggcccgatcc ttacccgtcc gttttacatt gccttgttct tgggcgggat ccacgggatg 60

caagcgggag ttaaaggagc gagaagcatc cggtatgaaa gagaacatca tggacctctg 120

gtgttcccat atcacagaag caacccg 147

<210> 45
<211> 215
<212> DNA
<213> EVEX C30

<400> 45
acggagagtg tgacatacac aactgtatca tctcacgagg taaaattaga tccataccag 60

atgactttcg tggattcact ctttccgggc ggtaaatgct cctcttcagt gtgtagcacc 120

atttatcatc aaggagtctg gataaacccg agtaacaact tagggttttg caaagatccg 180

gtcgatcatc aaggtcagtt gtacatggct ggtct 215

<210> 46
<211> 161
<212> DNA
<213> EVEX C30

<400> 46
ccacagatga gaagcctgaa catctctctg acgcattctc aaaatacaca gaatatttgt 60

ccaatgagag caaggggga g gaggaggatt tccaattcga acaggtggac tatggatttc 120

aagagtcacc tgaatccaat aaccctctgc aagagagctg g 161

<210> 47
<211> 237
<212> DNA
<213> EVEX C30

<400> 47
ggcatcacag aatctctcat ttctttattc caaaattcca aaaccattcg caacatcttc 60

aggaagaaat atgcgaaaga attggaactt agagtagtgc agtgtgaata cagatccatc 120

aatttgatgt tgagcctcgc agatcgatcc cacttagatg aaatgtggac atgctcggca 180

tctaaagcag acgaactccg aaccttatca tgggggacta caataattgg aacaact 237

<210> 48
<211> 192
<212> DNA
<213> EVEX C30

<400> 48
ggaattggga cagattgaca catcaggagc aaagttttca gatagggaag tccatcgggt 60

ttctgtttgg agatttgaca atgacaaaga attcacatgc tcaggattct tctattttcc 120

ctttatcaat tcaatacaag atcacagctg cggagtttct ggaaggaatt ctagatggga 180

tagtcaaggc tt 192

<210> 49
<211> 140
<212> DNA
<213> EVEX GG184

<400> 49
gaaggcactg aagaatgtca ctactctcat caacagtgcc aactatctga gaatggtggc 60

agtgattgac atgttctact accatttcaa gaacagtcaa gaaagagctg ttgtcaggat 120

tgccactctg agttcccgtc 140

<210> 50
<211> 147
<212> DNA
<213> EVEX GG184

<400> 50
ggcccgatcc ttacccgtcc gttttacatt gccttgttct tgggcgggat ccacgggatg 60

catgcgggat ttaaaggagc gagaagcatc cggtatgaaa gagaacatca tggacctctg 120

gtgttcccat atcacagaag caacccg 147

<210> 51
<211> 215
<212> DNA
<213> EVEX GG184

<400> 51
acggagagtg tgacatacac aactgtatca tctcacgagg taaaattaga tccataccag 60

atgactttcg tggattcact ctttccgggc ggtaaatgct cctcttcagt gtgtagcacc 120

atttatcatc aaggagtctg gataaaccct agtaacaatt taggattttg caaagatccg 180

gtcgatcatc aaggtcagtt gtacatggct ggtct 215

<210> 52
<211> 161
<212> DNA
<213> EVEX GG184

<400> 52
ccacagatga gaagcctgaa catctctctg acgcattctc aaaatacaca gaatatttgt 60

caaatgagag caagggggat gaggaggatt tccaatttga acaggtggac tatggatttc 120

aagagtcacc tgaatccaat aaccctctgc aagagagctg g 161

<210> 53
<211> 237
<212> DNA
<213> EVEX GG184

<400> 53
ggcatcacag aatctctcat ttctttattc caaaattcca aaaccattcg caacatattc 60

aggaagaaat atgcgaaaga attggaactt agagtagtgc agtgtgaata cagatccatc 120

aatttgatgc tgagcctcgc agatcaatcc cacttagatg aaatgtggac atgctcggca 180

tctaaagcag acgaactccg aaccttatca tgggggacta caataattgg aacaact 237

<210> 54
<211> 192
<212> DNA
<213> EVEX GG184

<400> 54
ggaattggga cagattgaca catcaggagc aaagttttca gatagggaag tccatcgggt 60

ttctgtttgg agatttgaca atgacaaaga attcacatgc ccaggattct tctattttcc 120

ctttatcaat tcaatacaag atcacagctg cggagtttct ggaaggaatt ctagatggga 180

tagtcaaggc tt 192

<210> 55
<211> 140
<212> DNA
<213> EVEX DF25/04

<400> 55
gaaggcactg aagaatgtca ctactctcat caacagtgcc aactatctga gaatggtggc 60

agtgattgac atgttctact accatttcaa gaacagtcaa gaaagagctg ttgtcaggat 120

tgccactctg agttcccgtc 140

<210> 56
<211> 147
<212> DNA
<213> EVEX DF25/04

<400> 56

ggcccgatcc ttacccgtcc attttacatt gccttgttct tgggcgggat ccacgggatg 60

caggcgggag ttaaaggagc gagaagcatc cggtatgaaa gagaacatca tggacctctg 120

gtgttcccat atcacagaag caacccg 147

<210> 57
<211> 215
<212> DNA
<213> EVEX DF25/04

<400> 57
acggagagtg tgacatacac aactgtatca tctcacgagg taaaattaga tccataccag 60

atgactttcg tggattcact tttcccgggc ggtaaatgct cctcttcagt gtgtagcacc 120

atttatcatc aaggagtctg gataagcccg agtgacaact tagggttttg caaagatcca 180

gtcgatcatc aaggtcagtt gtacatggct ggtct 215

<210> 58
<211> 161
<212> DNA
<213> EVEX DF25/04

<400> 58
ccacagatga gaagcctgaa catctctctg acgcattctc aaaatacaca gaatatttgt 60

ccaatgagag caaggggga gaagaggatt tccaattcga acaggtggac tatggatttc 120

aagagtcacc tgaatccaat aaccctctgc aagagagctg g 161

<210> 59
<211> 237
<212> DNA
<213> EVEX DF25/04

<400> 59
ggcatcacag aatctctcat ttctttattc caaaattcca aaaccattcg caacatcttc 60

aggaagaaat atgcaaaaga attggaactt agagtagtgc aatgtgaata cagatccatc 120

aatttgatgt tgagcctcgc agatcgatcc cacttagatg aaatgtggac atgctcggca 180

tctaaagcgg acgaacttcg aaccctatca tgggggacta caataattgg aacaact 237

<210> 60
<211> 192
<212> DNA
<213> EVEX DF25/04

<400> 60
ggaattggga cagattgaca catcaggagc aaagttttca gatagggaag tctatcgggt 60

ttctgtttgg agatttgaca atgacaaaga attcacatgc tcaggattct tctattttcc 120

ctttgtcaat tcaatacaag atcacagctg cggagtttct ggaaggaatt ctagatggga 180

tagtcaaggc tt 192

<210> 61
<211> 140
<212> DNA
<213> EVEX DK3545

<400> 61
gaaggcactg aagaatgtca ctactctcat caacagtgcc aactatctga aaatggtggc 60

agtgattgac atgttctact accatttcaa gaacagtcaa gaaagagccg ttgtcaggat 120

tgccactctg agttcccgcc 140

<210> 62
<211> 147
<212> DNA
<213> EVEX DK3545

<400> 62
ggcccgatcc ttacccgtcc gttttacatt gccttgttct tgggcgggat ccacgggatg 60

caggcggggg ttaaaggagc gagaagcatc cggtatgaaa gagaacatca tggacctctg 120

gtgttcccat atcacagaag caacccg 147

<210> 63
<211> 215
<212> DNA
<213> EVEX DK3545

<400> 63
acggagagtg tgacatacac aactgtatca tctcacgagg taaaactaga tccataccag 60

atgactttcg tggattcact ctttccgggt ggtaaatgct cctcttcggt gtgtagcacc 120

atctatcatc aaggagtctg gataaacccg agtcacaact tagggttttg caaagatcca 180

gtcgatcatc aaggtcagtt gtacatggct ggtct 215

<210> 64
<211> 161
<212> DNA
<213> EVEX DK3545

<400> 64
ccacagatga gaagcctgaa catctctcgg acgcattttc aaaatacaca gaatatttgt 60

caaatgagag caaggtggag gaggaggatt tccaattcga acaggtggaa tatggatttc 120

aagaatcacc tgaatccaat aaccctctgc aagagagctg g 161

<210> 65
<211> 237
<212> DNA
<213> EVEX DK3545

<400> 65
ggcatcacag aatctctcat ttctttattc caaaattcca aaaccattcg caacatcttc 60

aggaagaaat atgcaaaaga attggaactt agagtagtgc agtgtgaata cagatccatc 120

aatttgatgt tgagcctcgc agatcgatcc catttagatg aaatgtggac atgctcggca 180

tctaaagcag atgaactccg aaccttatca tgggggacta caataattgg aacaact 237

<210> 66
<211> 192
<212> DNA
<213> EVEX DK3545

<400> 66
ggaattggga cagattgaca catcaggagc aaagttttca gatagggaag tccatcgggt 60

ttctgtttgg agatttgaca atgacaaaga attcacatgc tcaggattct tctattttcc 120

ctttatcaat tcaatacaag atcacagctg cggagtttct ggaaggaatt ctagatggga 180

tagtcaaggc tt 192

<210> 67
<211> 140
<212> DNA
<213> EVEX DK3631

<400> 67
gaaggcactg aagaatgtca ctactctcat caacagtgcc aactatctga aaatggtggc 60

agtgattgac atgttctact accatttcaa gaacagtcaa gaaagagctg ttgtcaggat 120

tgccactctg agttcccgtc 140

<210> 68
<211> 147
<212> DNA
<213> EVEX DK3631

<400> 68
ggcccgatcc tgacccgtcc gttttacatt gccttgttct tgggcgggat tcacgggatg 60

caggcggggg ttaaaggagc gagaagcatc cggtatgaaa gagaacatca tggacctctg 120

gtgttcccat atcacagaag caacccg 147

<210> 69
<211> 215
<212> DNA
<213> EVEX DK3631

<400> 69
acggagagtg tgacatacac aactgtatca tctcacgagg taaaattaga tccataccag 60

atgactttcg tggattcact ctttccgggc ggtaaatgct cctcttcggt gtgtagcacc 120

atctatcatc aaggagtctg gataaacccg agtcacaact tagggttttg caaagatcca 180

gtcgatcatc aaggtcagtt gtacatggct ggtct 215

<210> 70
<211> 161
<212> DNA
<213> EVEX DK3631

<400> 70
ccacagatga gaagcctgaa catctctctg acgcattttc aaaatacaca gaatatttgt 60

caaatgagag caagggggag gaggaggatt tccaattcga acaggtggaa tatggatttc 120

aagagtcacc tgaatccaat aaccctctgc aagagagctg g 161

<210> 71
<211> 237
<212> DNA
<213> EVEX DK3631

<400> 71
ggcatcacag aatctctcat ttctttattc caaaattcca aaaccattcg caacatcttc 60

aggaagaaat atgcgaaaga attggaactt agagtagtgc agtgtgaata cagatccatc 120

aatttgatgt tgagcctcgc agatcgatcc cacttagatg aaatgtggac atgctcggca 180

tctaaagcag atgaactccg aaccttatca tgggggacta caataattgg aacaact 237

<210> 72
<211> 192
<212> DNA
<213> EVEX DK3631

<400> 72
ggaactggga cagattgaca catcaggaac aaagttttca gatagggaag tctatcgggt 60

ttctgtttgg agatttgaca atgacaaaga attcacatgc tcaggattct tctattttcc 120

ctttatcaat tcaatacaag atcacagctg cggagtttct ggaaggaatt ctagatggga 180

tagtcaaggc tt 192

<210> 73
<211> 140
<212> DNA
<213> EVEX DK5743

<400> 73
gaaggcactg aagaatgtca ctactctcat caacagtgcc aactatctga aaatggtggc 60

agtgattgac atgttctact accatttcaa gaacagtcaa gaaagagctg ttgtcaggat 120

tgccactctg agttcccgtc 140

<210> 74
<211> 147
<212> DNA
<213> EVEX DK5743

<400> 74

ggcccgatcc ttacccgtcc gttttacatt gccttgttct tgggcgggat ccacgggatg 60

caagcgggag ttaaaggagc gagaagcatc ctgtatgaaa gagaacatca tggacctctg 120

gtgttcccat atcacagaag caacccg 147

<210> 75
<211> 215
<212> DNA
<213> EVEX DK5743

<400> 75
acggagagtg tgacatacac aactgtatca tctcccgagg taaaattaga tccataccag 60

atgactttcg tggattcact ctttccgggc ggtaaatgct cctcttcagt gtgtagcacc 120

atttatcatc aaggagtctg gataaacccg agcaataact tagggttttg caaagatccg 180

gtcgatcatc aaggtcagtt gtacatggct ggtct 215

<210> 76
<211> 161
<212> DNA
<213> EVEX DK5743

<400> 76
ccacagatga gaagcctgaa catctctctg acgcattctc aaaatacaca gaatatttgt 60

ccaatgagag caagggggag gaggaggatt tccaattcga acaggtggac tatggatttc 120

aagagtcacc tgaatccaat aaccctctgc aagagagctg g 161

<210> 77
<211> 237
<212> DNA
<213> EVEX DK5743

<400> 77
ggcatcacag aatctctcat ttctttattc caaaattcca aaaccattcg caacatcttc 60

aggaagaaat atgcgaaaga attggaactt agagtagtgc agtgtgaata cagatccatc 120

aatttgatgt tgagcctcgc agatcgatcc cacttagatg aaatgtggac atgctcggca 180

tctaaagcag acgaactccg aaccttatca tgggggacta caataattgg aacaact 237

<210> 78
<211> 192
<212> DNA
<213> EVEX DK5743

<400> 78
ggaattggga cagattgaca catcaggagc aaagttttca gatagggaag tccattgggt 60

ttctgtttgg agatttgaca atgacaaaga attcacatgc tcaggattct tctattttcc 120

ctttatcaat tcaatacaag atcacagctg cggagtttct ggaaggaatt ctagatggga 180

tagtcaaggc tt 192

<210> 79
<211> 140
<212> DNA
<213> EVA

<400> 79
gaaggcactg aagaatgtca ctactcttat taacagtgca aactatctga gaatggtggc 60

agtgattgat atgttctact accatttcaa gaacagtcaa gaaagagctg ttgtcagaat 120

tgccactctg agttcccgtc 140

<210> 80
<211> 147
<212> DNA
<213> EVA

<400> 80
ggcccgatcc ttacccgtcc tttttacata gccttgtttt tgggcgggat acacgggatg 60

caggcaggag tcaaaggagc gagaagcatc aggtatgaga gagaacatca gggacctctg 120

gtgttcccat atcacagaag caacccg 147

<210> 81
<211> 215
<212> DNA
<213> EVA

<400> 81
acggagagtg tgacatacac aactgtatca tctcacgagg taaaactaga tccataccag 60

atgactttcg tggattcact ctttccgggc ggtaaatgct cctcttcagt gtgtaacact 120

atctatcatc aaggagtctg gataaacctg aatgacaact tagggttttg cagagaacca 180

gtcgatcatc aaggtcagtt gtacatggct ggtct 215

<210> 82
<211> 161
<212> DNA
<213> EVA

<400> 82
ccacagatga gaagcctgaa catctctctg acgcattctc aaagtataca gaatatctgt 60

ccaatgagag caaggtggag gaggaggatt tgcaattcga acaggtggac tatggatttc 120

aagaatcacc ggagtccaat aaccctctgc aagagagctg g 161

<210> 83
<211> 237
<212> DNA
<213> EVA

<400> 83
ggcatcacag aatctctcat ttctttattc caaaattcta aaaccattcg caacatcttt 60

```
agaaagaaat atgcaaaaga attagaactt agagtagtgc agtgtgagta cagatccatc    120

aatctgatgt tgagtctcgc agatcgatcc cacctggatg aaatgtggac atgttcagca    180

tccaaagcgg atgaactccg aacactatct tgggggacta caataattgg aacaact       237


<210>  84
<211>  192
<212>  DNA
<213>  EVA

<400>  84
ggaattggga cagattgaca catcaggaac agagttttca gatagggaaa tctatcgggt     60

ttctatttgg agatttaaca atgaccaaga attcacatgc tcaagactct tctattttcc    120

cactgtcaat tcaatacaag atcacagccg cggagttcct agaaggaatt ctagatggga    180

tagtcaaggc tt                                                         192
```